# EUROPEAN PATENT SPECIFICATION

(11) **EP 4 335 923 B1**
(45) Date of publication and mention of the grant of the patent: **09.12.2026**
(21) Application number: 23210648.4
(22) Date of filing: 29.09.2017
(51) Int. Cl.: C12N 15/113

(54) **COMPOUNDS AND METHODS FOR REDUCING TAU EXPRESSION**
VERBINDUNGEN UND VERFAHREN ZUR REDUZIERUNG DER TAU-EXPRESSION
COMPOSÉS ET PROCÉDÉS POUR RÉDUIRE L'EXPRESSION DE TAU

(30) Priority: 29.09.2016 US 201662401723 P; 25.01.2017 US 201762450469 P
(43) Date of publication of application: 13.03.2024
(62) Document number(s) of the earlier application(s) in accordance with Art. 76 EPC: 17857565.0 / 3 519 572
(73) Proprietor: Biogen MA Inc., Cambridge, MA 02142 (US)
(72) Inventor: KORDASIEWICZ, Holly, Carlsbad, CA 92010 (US)
(74) Representative: Carpmaels & Ransford LLP

(56) References cited:
- US-A1- 2016 145 617

## Description

### Field

Provided are compounds for use in methods for reducing the amount or activity of Tau mRNA in a cell or human subject, and in certain instances reducing the amount of Tau protein in a cell or human subject. Such compounds for use in methods are useful to ameliorate at least one symptom of a neurodegenerative disease. Such symptoms include loss of memory, loss of motor function, and increase in the number and/or volume of neurofibrillary inclusions. Such neurodegenerative diseases include tauopathies, Alzheimer's Disease, Fronto-temporal Dementia (FTD), FTDP-17, Progressive Supranuclear Palsy (PSP), Chronic Traumatic Encephalopathy (CTE), Corticobasal Ganglionic Degeneration (CBD), Epilepsy, and Dravet's Syndrome.

### Background

The primary function of Tau is to bind to and stabilize microtubules, which are important structural components of the cytoskeleton involved in mitosis, cytokinesis, and vesicular transport. Tau is found in multiple tissues, but is particularly abundant in axons of neurons. In humans, there are six isoforms of Tau that are generated by alternative splicing of exons 2, 3, and 10. Splicing of exons 2 and 3 at the N-terminus of the protein leads to inclusion of zero, one, or two 29 amino acid acidic domains and is termed 0N, 1N, or 2N Tau respectively. The influence of these domains on Tau function is not fully clear, though may play a role in interactions with the plasma membrane. Inclusion of exon 10 at the C-terminus leads to inclusion of the microtubule binding domain encoded by exon 10. Since there are 3 microtubule binding domains elsewhere in Tau, this Tau isoform (with exon 10 included) is termed 4R Tau, where 'R' refers to the number of repeats of microtubule binding domains. Tau without exon 10 is termed 3R Tau. Since more microtubule binding domains (4R compared with 3R) increases the binding to microtubules, 4R Tau presumably significantly increases microtubule binding and assembly. The ratio of 3R/4R Tau is developmentally regulated, with fetal tissues expressing exclusively 3R Tau and adult human tissues expressing approximately equal levels of 3R/4R Tau. Deviations from the normal ratio of 3R/4R Tau are characteristic of neurodegenerative FTD Tauopathies. It is not known how changing the 3R/4R Tau ratio at a later stage in the adult animal will affect Tau pathogenesis.

Serine-threonine directed phosphorylation regulates the microtubule binding ability of Tau. Hyperphosphorylation promotes detachment of Tau from microtubules. Other post translational modifications of Tau have been described; however the significance of these is unclear. Phosphorylation of Tau is also developmentally regulated with higher phosphorylation in fetal tissues and much lower phosphorylation in the adult. One characteristic of neurodegenerative disorders is aberrantly increased Tau phosphorylation.
The microtubule network is involved in many important processes within the cell including structural integrity needed for maintaining morphology of cells and operating transport machinery. Since binding of Tau to microtubules stabilizes microtubules, Tau is likely to be a key mediator of some of these processes and disruption of normal Tau in neurodegenerative diseases may disrupt some of these key cellular processes. One of the early indicators that Tau may be important in neurodegenerative syndromes was the recognition that Tau is a key component of neurofibrillary inclusions in Alzheimer's disease. In fact, neurofibrillary inclusions are aggregates of hyperphosphorylated Tau protein. Along with amyloid beta containing plaques, neurofibrillary inclusions are a hallmark of Alzheimer's disease and correlate significantly with cognitive impairment. 95% of Tau accumulations in AD are found in neuronal processes and is termed neuritic dystrophy. The process(es) whereby this microtubule associated protein becomes disengaged from microtubules and forms accumulations of proteins and how this relates to neuronal toxicity is not well understood.

Neuronal Tau inclusions are a pathological characteristic of not only Alzheimer's disease, but also a subset of Fronto-temporal dementia (FTD), PSP, and CBD. The link between Tau and neurodegeneration was solidified by the discovery that mutations in the Tau gene cause a subset of FTD. These genetic data have also highlighted the importance of the 3R:4R ratio of Tau. Many of the Tau mutations that cause FTD lead to a change in Tau splicing which leads to preferential inclusion of exon 10, and thus to increased 4R Tau. The overall Tau levels are normal. Whether the Tau isoform change or the amino acid change or both cause neurodegeneration remains unknown. Recent data suggest that PSP may also be associated with an increased 4R:3R Tau ratio.

To help understand the influence of Tau ratios on neurodegeneration, a mouse model based on one of the splicing Tau mutations (N279K) has been generated using a minigene that includes the Tau promoter and the flanking intronic sequences of exon 10. As in humans, these mice demonstrate increased levels of 4R Tau compared with transgenics expressing WT Tau and develop behavioral and motor abnormalities as well as accumulations of aggregated Tau in the brain and spinal cord.

Tau protein has been associated with multiple diseases of the brain including Alzheimer's disease, FTD, PSP, CBD, dementia pugilistica, parkinsonism linked to chromosome, Lytico-Bodig disease, tangle-predominant dementia, ganglioglioma, gangliocytoma, meningioangiomatosis, subacute sclerosing panencephalitis, lead encephalopathy, tuberous sclerosis, Hallervorden-Spatz disease, Pick's disease, argyrophilic grain disease, corticobasal degeneration or fronto-temporal lobar degeneration and others. Tau-associated disorders such as AD are the most common cause of dementia in the elderly. AD affects an estimated 15 million people worldwide and 40% of the population above 85 years of age. AD is characterized by two pathological hallmarks: Tau neurofibrillary inclusions (NFT) and amyloid-β (Aβ) plaques.

There is currently a lack of acceptable options for treating such neurodegenerative diseases. It is therefore an object herein to provide modified oligonucleotides, salts, and compounds for use in treating such diseases in a human subject in need thereof.

### Summary of the Invention

The present invention provides a modified oligonucleotide according to the following formula: 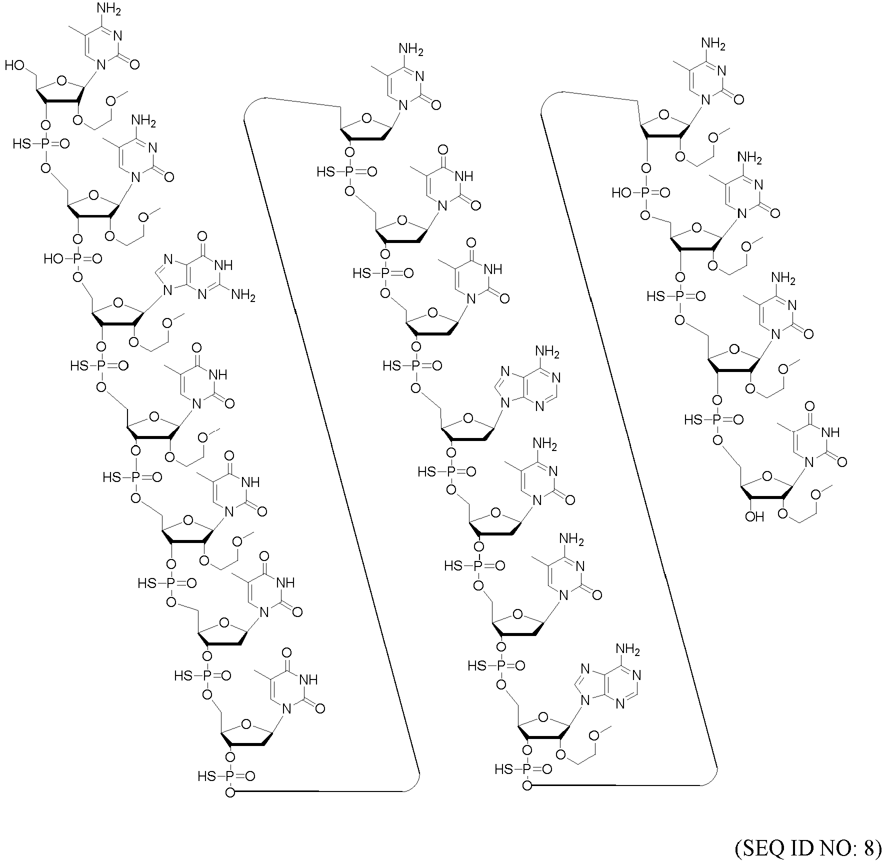 or a salt thereof, for use in a method of treating a neurodegenerative disease associated with tau in a human subject in need thereof.

The invention also provides a modified oligonucleotide for use in a method of treating a neurodegenerative disease associated with tau in a human subject in need thereof, wherein the modified oligonucleotide is a gapmer consisting of a 5' wing segment, a central gap segment, and a 3' wing segment, wherein:
the 5' wing segment consists of five 2'-MOE nucleosides,
the central gap segment consists of eight 2'-deoxynucleosides, and
the 3' wing segment consists of five 2'-MOE nucleosides;
wherein the modified oligonucleotide has the nucleobase sequence 5'-CCGTTTTCTTACCACCCT-3' (SEQ ID NO: 8), wherein each cytosine is a 5-methylcytosine; and wherein the internucleoside linkages of the modified oligonucleotide are, from 5' to 3', sossssssssssssoss, wherein each s is a phosphorothioate linkage and each o is a phosphodiester linkage.

The invention also provides a compound comprising a modified oligonucleotide for use in a method of treating a neurodegenerative disease associated with tau in a human subject in need thereof, wherein the modified oligonucleotide is a gapmer consisting of a 5' wing segment, a central gap segment, and a 3' wing segment, wherein:
the 5' wing segment consists of five 2'-MOE nucleosides,
the central gap segment consists of eight 2'-deoxynucleosides, and
the 3' wing segment consists of five 2'-MOE nucleosides;
wherein the modified oligonucleotide has the nucleobase sequence 5'-CCGTTTTCTTACCACCCT-3' (SEQ ID NO: 8), wherein each cytosine is a 5-methylcytosine; and wherein the internucleoside linkages of the modified oligonucleotide are, from 5' to 3', sossssssssssssoss, wherein each s is a phosphorothioate linkage and each o is a phosphodiester linkage.

Further embodiments of the invention are defined in the appended claims.
Provided herein are therefore compounds and their use in methods of treating a neurodegenerative disease associated with tau in a human subject in need thereof. In certain embodiments, the modified oligonucleotide, salt, or compound for use reduces the amount or activity of Tau mRNA, and in certain embodiments reducing the amount of Tau protein in a cell or human subject. The human subject has a neurodegenerative disease associated with tau. In certain embodiments, the human subject has a tauopathy, Alzheimer's Disease, Fronto-temporal Dementia (FTD), FTDP-17, Progressive Supranuclear Palsy (PSP), Chronic Traumatic Encephalopathy (CTE), Corticobasal Ganglionic Degeneration (CBD), Epilepsy, or Dravet's Syndrome. In certain embodiments, compounds useful for reducing expression of Tau mRNA are oligomeric compounds. In certain embodiments, Compound No. 814907 is useful for reducing expression of Tau mRNA and/or Tau protein.

Also disclosed herein are methods useful for preventing or ameliorating at least one symptom of a neurodegenerative disease. In certain instances, such symptoms are loss of memory, loss of motor function, and increase in the number and/or volume of neurofibrillary inclusions. In certain instances, prevention or amelioration results in maintining or improving memory, maintaining or improving motor function, and/or maintenance or reduction in the number and/or volume of neurofibrillary inclusions. In certain instances, such prevention or amelioration of symptoms is the decrease in the rate of progression or delay in onest of such symptoms.

### Detailed Description of the Invention

It is to be understood that both the foregoing general description and the following detailed description are exemplary and explanatory only and are not restrictive. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature used in connection with, and the procedures and techniques of, analytical chemistry, synthetic organic chemistry, and medicinal and pharmaceutical chemistry described herein are those well known and commonly used in the art.

Unless otherwise indicated, the following terms have the following meanings:

### DEFINITIONS

As used herein, "2'-deoxynucleoside" means a nucleoside comprising 2'-H(H) furanosyl sugar moiety, as found in naturally occurring deoxyribonucleic acids (DNA). In certain embodiments, a 2'-deoxynucleoside may comprise a modified nucleobase or may comprise an RNA nucleobase (uracil).

As used herein, "2'-substituted nucleoside" means a nucleoside comprising a 2'-substituted sugar moiety. As used herein, "2'-substituted" in reference to a sugar moiety means a sugar moiety comprising at least one 2'-substituent group other than H or OH.

As used herein, "administering" means providing a pharmaceutical agent to an animal.

As used herein, "animal" means a human or non-human animal.

As used herein, "antisense activity" means any detectable and/or measurable change attributable to the hybridization of an antisense compound to its target nucleic acid. In certain embodiments, antisense activity is a decrease in the amount or expression of a target nucleic acid or protein encoded by such target nucleic acid compared to target nucleic acid levels or target protein levels in the absence of the antisense compound.

As used herein, "antisense compound" means an oligomeric compound or oligomeric duplex capable of achieving at least one antisense activity.

As used herein, "ameliorate" in reference to a treatment means improvement in at least one symptom relative to the same symptom in the absence of the treatment. In certain embodiments, amelioration is the reduction in the severity or frequency of a symptom or the delayed onset or slowing of progression in the severity or frequency of a symptom. In certain embodiments, the symptom is loss of memory, loss of motor function, or increase in the number and/or volume of neurofibrillary inclusions. In certain embodiments, amelioration of these symptoms results in maintining or improving memory, maintaining or improving motor function, and/or maintenance or reduction in the number and/or volume of neurofibrillary inclusions.

As used herein, "at least one symptom of a neurodegenerative disease" includes loss of memory, loss of motor function, or increase in the number and/or volume of neurofibrillary inclusions.

As used herein, "bicyclic nucleoside" or "BNA" means a nucleoside comprising a bicyclic sugar moiety. As used herein, "bicyclic sugar" or "bicyclic sugar moiety" means a modified sugar moiety comprising two rings, wherein the second ring is formed via a bridge connecting two of the atoms in the first ring thereby forming a bicyclic structure. In certain embodiments, the first ring of the bicyclic sugar moiety is a furanosyl moiety. In certain embodiments, the bicyclic sugar moiety does not comprise a furanosyl moiety.

As used herein, "chirally enriched population" means a plurality of molecules of identical molecular formula, wherein the number or percentage of molecules within the population that contain a particular stereochemical configuration at a particular chiral center is greater than the number or percentage of molecules expected to contain the same particular stereochemical configuration at the same particular chiral center within the population if the particular chiral center were stereorandom. Chirally enriched populations of molecules having multiple chiral centers within each molecule may contain one or more sterorandom chiral centers. In certain embodiments, the molecules are modified oligonucleotides. In certain embodiments, the molecules are compounds comprising modified oligonucleotides.

As used herein, "cleavable moiety" means a bond or group of atoms that is cleaved under physiological conditions, for example, inside a cell, an animal, or a human.

As used herein, "complementary" in reference to an oligonucleotide means that at least 70% of the nucleobases of the oligonucleotide or one or more regions thereof and the nucleobases of another nucleic acid or one or more regions thereof are capable of hydrogen bonding with one another when the nucleobase sequence of the oligonucleotide and the other nucleic acid are aligned in opposing directions. Complementary nucleobases means nucleobases that are capable of forming hydrogen bonds with one another. Complementary nucleobase pairs include adenine (A) and thymine (T), adenine (A) and uracil (U), cytosine (C) and guanine (G), 5-methylcytosine (mC) and guanine (G). Complementary oligonucleotides and/or nucleic acids need not have nucleobase complementarity at each nucleoside. Rather, some mismatches are tolerated. As used herein, "fully complementary" or "100% complementary" in reference to oligonucleotides means that oligonucleotides are complementary to another oligonucleotide or nucleic acid at each nucleoside of the oligonucleotide.

As used herein, "conjugate group" means a group of atoms that is directly or indirectly attached to an oligonucleotide. Conjugate groups include a conjugate moiety and a conjugate linker that attaches the conjugate moiety to the oligonucleotide.

As used herein, "conjugate linker" means a group of atoms comprising at least one bond that connects a conjugate moiety to an oligonucleotide.

As used herein, "conjugate moiety" means a group of atoms that is attached to an oligonucleotide via a conjugate linker.

As used herein, "contiguous" in the context of an oligonucleotide refers to nucleosides, nucleobases, sugar moieties, or internucleoside linkages that are immediately adjacent to each other. For example, "contiguous nucleobases" means nucleobases that are immediately adjacent to each other in a sequence.

As used herein, "gapmer" means a modified oligonucleotide comprising an internal region having a plurality of nucleosides that support RNase H cleavage positioned between external regions having one or more nucleosides, wherein the nucleosides comprising the internal region are chemically distinct from the nucleoside or nucleosides comprising the external regions. The internal region may be referred to as the "gap" and the external regions may be referred to as the "wings." Unless otherwise indicated, "gapmer" refers to a sugar motif. Unless otherwise indicated, the sugar moieties of the nucleosides of the gap of a gapmer are unmodified 2'-deoxyfuranosyl. Thus, the term "MOE gapmer" indicates a gapmer having a sugar motif of 2'-MOE nucleosides in both wings and a gap of 2'-deoxynucleosides. Unless otherwise indicated, a MOE gapmer may comprise one or more modified internucleoside linkages and/or modified nucleobases and such modifications do not necessarily follow the gapmer pattern of the sugar modifications.

As used herein, "hybridization" means the pairing or annealing of complementary oligonucleotides and/or nucleic acids. While not limited to a particular mechanism, the most common mechanism of hybridization involves hydrogen bonding, which may be Watson-Crick, Hoogsteen or reversed Hoogsteen hydrogen bonding, between complementary nucleobases.

As used herein, the term "internucleoside linkage" is the covalent linkage between adjacent nucleosides in an oligonucleotide. As used herein "modified internucleoside linkage" means any internucleoside linkage other than a phosphodiester internucleoside linkage. "Phosphorothioate linkage" is a modified internucleoside linkage in which one of the non-bridging oxygen atoms of a phosphodiester internucleoside linkage is replaced with a sulfur atom.

As used herein, "linker-nucleoside" means a nucleoside that links, either directly or indirectly, an oligonucleotide to a conjugate moiety. Linker-nucleosides are located within the conjugate linker of an oligomeric compound. Linker-nucleosides are not considered part of the oligonucleotide portion of an oligomeric compound even if they are contiguous with the oligonucleotide.

As used herein, "non-bicyclic modified sugar moiety" means a modified sugar moiety that comprises a modification, such as a substitutent, that does not form a bridge between two atoms of the sugar to form a second ring.

As used herein, "mismatch" or "non-complementary" means a nucleobase of a first oligonucleotide that is not complementary with the corresponding nucleobase of a second oligonucleotide or target nucleic acid when the first and second oligomeric compound are aligned.

As used herein, "MOE" means methoxyethyl. "2'-MOE" means a -OCH₂CH₂OCH₃ group at the 2' position of a furanosyl ring.

As used herein, "motif" means the pattern of unmodified and/or modified sugar moieties, nucleobases, and/or internucleoside linkages, in an oligonucleotide.

As used herein, "mRNA" means an RNA transcript that encodes a protein and includes pre-mRNA and mature mRNA unless otherwise specified.

As used herein, "nucleobase" means an unmodified nucleobase or a modified nucleobase. As used herein an "unmodified nucleobase" is adenine (A), thymine (T), cytosine (C), uracil (U), and guanine (G). As used herein, a "modified nucleobase" is a group of atoms other than unmodified A, T, C, U, or G capable of pairing with at least one unmodified nucleobase. A "5-methylcytosine" is a modified nucleobase. A universal base is a modified nucleobase that can pair with any one of the five unmodified nucleobases. As used herein, "nucleobase sequence" means the order of contiguous nucleobases in a nucleic acid or oligonucleotide independent of any sugar or internucleoside linkage modification.

As used herein, "nucleoside" means a compound comprising a nucleobase and a sugar moiety. The nucleobase and sugar moiety are each, independently, unmodified or modified. As used herein, "modified nucleoside" means a nucleoside comprising a modified nucleobase and/or a modified sugar moiety. Modified nucleosides include abasic nucleosides, which lack a nucleobase. "Linked nucleosides" are nucleosides that are connected in a contiuous sequence (i.e., no additional nucleosides are presented between those that are linked).

As used herein, "oligomeric compound" means an oligonucleotide and optionally one or more additional features, such as a conjugate group or terminal group. An oligomeric compound may be paired with a second oligomeric compound that is complementary to the first oligomeric compound or may be unpaired. A "singled-stranded oligomeric compound" is an unpaired oligomeric compound. The term "oligomeric duplex" means a duplex formed by two oligomeric compounds having complementary nucleobase sequences. Each oligomeric compound of an oligomeric duplex may be refered to as a "duplexed oligomeric compound."

As used herein, "oligonucleotide" means a strand of linked nucleosides connected via internucleoside linkages, wherein each nucleoside and internucleoside linkage may be modified or unmodified. Unless otherwise indicated, oligonucleotides consist of 8-50 linked nucleosides. As used herein, "modified oligonucleotide" means an oligonucleotide, wherein at least one nucleoside or internucleoside linkage is modified. As used herein, "unmodified oligonucleotide" means an oligonucleotide that does not comprise any nucleoside modifications or internucleoside modifications.

As used herein, "pharmaceutically acceptable carrier or diluent" means any substance suitable for use in administering to an animal. Certain such carriers enable pharmaceutical compositions to be formulated as, for example, tablets, pills, dragees, capsules, liquids, gels, syrups, slurries, suspension and lozenges for the oral ingestion by a subject. In certain embodiments, a pharmaceutically acceptable carrier or diluent is sterile water; sterile saline; or sterile buffer solution.

As used herein "pharmaceutically acceptable salts" means physiologically and pharmaceutically acceptable salts of compounds, such as oligomeric compounds, i.e., salts that retain the desired biological activity of the parent compound and do not impart undesired toxicological effects thereto.

As used herein "pharmaceutical composition" means a mixture of substances suitable for administering to a subject. For example, a pharmaceutical composition may comprise an antisense compound and a sterile aqueous solution. In certain embodiments, a pharmaceutical composition shows activity in free uptake assay in certain cell lines.

As used herein, "phosphorus moiety" means a group of atoms comprising a phosphorus atom. In certain embodiments, a phosphorus moiety comprises a mono-, di-, or tri-phosphate, or phosphorothioate.

As used herein "prodrug" means a therapeutic agent in a form outside the body that is converted to a different form within an animal or cells thereof. Typically conversion of a prodrug within the animal is facilitated by the action of an enzymes (e.g., endogenous or viral enzyme) or chemicals present in cells or tissues and/or by physiologic conditions.

As used herein, "reducing or inhibiting the amount or activity" refers to a reduction or blockade of the transcriptional expression or activity relative to the transcriptional expression or activity in an untreated or control sample and does not necessarily indicate a total elimination of transcriptional expression or activity.

As used herein, "RNAi compound" means an antisense compound that acts, at least in part, through RISC or Ago2 to modulate a target nucleic acid and/or protein encoded by a target nucleic acid. RNAi compounds include, but are not limited to double-stranded siRNA, single-stranded RNA (ssRNA), and microRNA, including microRNA mimics. In certain embodiments, an RNAi compound modulates the amount, activity, and/or splicing of a target nucleic acid. The term RNAi compound excludes antisense compounds that act through RNase H.

As used herein, "Self-complementary" in reference to an oligonucleotide means an oligonucleotide that at least partially hybridizes to itself.

As used herein, "standard cell assay" means the assay described in Example 1 and reasonable variations thereof.

As used herein, "stereorandom chiral center" in the context of a population of molecules of identical molecular formula means a chiral center having a random stereochemical configuration. For example, in a population of molecules comprising a stereorandom chiral center, the number of molecules having the (S) configuration of the stereorandom chiral center may be but is not necessarily the same as the number of molecules having the (R) configuration of the stereorandom chiral center. The stereochemical configuration of a chiral center is considered random when it is the result of a synthetic method that is not designed to control the stereochemical configuration. In certain embodiments, a stereorandom chiral center is a stereorandom phosphorothioate internucleoside linkage.

As used herein, "sugar moiety" means an unmodified sugar moiety or a modified sugar moiety. As used herein, "unmodified sugar moiety" means a 2'-OH(H) furanosyl moiety, as found in RNA (an "unmodified RNA sugar moiety"), or a 2'-H(H) moiety, as found in DNA (an "unmodified DNA sugar moiety"). Unmodified sugar moieties have one hydrogen at each of the 1', 3', and 4' positions, an oxygen at the 3' position, and two hydrogens at the 5' position. As used herein, "modified sugar moiety" or "modified sugar" means a modified furanosyl sugar moiety or a sugar surrogate. As used herein, modified furanosyl sugar moiety means a furanosyl sugar comprising a non-hydrogen substituent in place of at least one hydrogen of an unmodified sugar moiety. In certain embodiments, a modified furanosyl sugar moiety is a 2'-substituted sugar moiety. Such modified furanosyl sugar moieties include bicyclic sugars and non-bicyclic sugars.

As used herein, "sugar surrogate" means a modified sugar moiety having other than a furanosyl moiety that can link a nucleobase to another group, such as an internucleoside linkage, conjugate group, or terminal group in an oligonucleotide. Modified nucleosides comprising sugar surrogates can be incorporated into one or more positions within an oligonucleotide and such oligonucleotides are capable of hybridizing to complementary oligomeric compounds or nucleic acids.

As used herein, "target nucleic acid" and "target RNA" mean a nucleic acid that an antisense compound is designed to affect.

As used herein, "target region" means a portion of a target nucleic acid to which an oligomeric compound is designed to hybridize.

As used herein, "terminal group" means a chemical group or group of atoms that is covalently linked to a terminus of an oligonucleotide.

As used herein, "therapeutically effective amount" means an amount of a pharmaceutical agent that provides a therapeutic benefit to an animal. For example, a therapeutically effective amount improves a symptom of a disease.

### I. Certain Oligonucleotides

In certain embodiments, provided herein are oligonucleotides for use, which consist of linked nucleosides. Oligonucleotides may be unmodified oligonucleotides (RNA or DNA) or may be modified oligonucleotides. Modified oligonucleotides comprise at least one modification relative to unmodified RNA or DNA. That is, modified oligonucleotides comprise at least one modified nucleoside (comprising a modified sugar moiety and/or a modified nucleobase) and/or at least one modified internucleoside linkage.

### A. Certain Modified Nucleosides

Modified nucleosides comprise a modified sugar moiety or a modified nucleobase or both a modifed sugar moiety and a modified nucleobase.

### 1. Certain Sugar Moieties

In certain embodiments, modified sugar moieties are non-bicyclic modified sugar moieties. In certain instances, modified sugar moieties are bicyclic or tricyclic sugar moieties. In certain instances, modified sugar moieties are sugar surrogates. Such sugar surrogates may comprise one or more substitutions corresponding to those of other types of modified sugar moieties.

In certain embodiments, modified sugar moieties are non-bicyclic modified sugar moieties comprising a furanosyl ring with one or more substituent groups none of which bridges two atoms of the furanosyl ring to form a bicyclic structure. Such non bridging substituents may be at any position of the furanosyl, including but not limited to substituents at the 2', 4', and/or 5' positions. In certain instances one or more non-bridging substituent of non-bicyclic modified sugar moieties is branched. Examples of 2'-substituent groups suitable for non-bicyclic modified sugar moieties include but are not limited to: 2'-F, 2'-OCH₃ ("OMe" or "O-methyl"), and 2'-O(CH₂)₂OCH₃ ("MOE"). In certaininstances, 2'-substituent groups are selected from among: halo, allyl, amino, azido, SH, CN, OCN, CF₃, OCF₃, O-C₁-C₁₀ alkoxy, O-C₁-C₁₀ substituted alkoxy, O-C₁-C₁₀ alkyl, O-C₁-C₁₀ substituted alkyl, S-alkyl, N(Rₘ)-alkyl, O-alkenyl, S-alkenyl, N(Rₘ)-alkenyl, O-alkynyl, S-alkynyl, N(Rₘ)-alkynyl, O-alkylenyl-O-alkyl, alkynyl, alkaryl, aralkyl, O-alkaryl, O-aralkyl, O(CH₂)₂SCH₃, O(CH₂)₂ON(Rₘ)(Rₙ) or OCH₂C(=O)-N(Rₘ)(Rₙ), where each Rₘ and Rₙ is, independently, H, an amino protecting group, or substituted or unsubstituted C₁-C₁₀ alkyl, and the 2'-substituent groups described in Cook et al., U.S. 6,531,584; Cook et al., U.S. 5,859,221; and Cook et al., U.S. 6,005,087. Certain instances of these 2'-substituent groups can be further substituted with one or more substituent groups independently selected from among: hydroxyl, amino, alkoxy, carboxy, benzyl, phenyl, nitro (NO₂), thiol, thioalkoxy, thioalkyl, halogen, alkyl, aryl, alkenyl and alkynyl. Examples of 4'-substituent groups suitable for non-bicyclic modified sugar moieties include but are not limited to alkoxy (*e.g.,* methoxy), alkyl, and those described in Manoharan et al., WO 2015/106128. Examples of 5'-substituent groups suitable for non-bicyclic modified sugar moieties include but are not limited to: 5'-methyl (R or S), 5'-vinyl, and 5'-methoxy. In certain instances, non-bicyclic modified sugar moieties comprise more than one non-bridging sugar substituent, for example, 2'-F-5'-methyl sugar moieties and the modified sugar moieties and modified nucleosides described in Migawa et al., WO 2008/101157 and Rajeev et al., US2013/0203836.).

In certain instances, a 2'-substituted non-bicyclic modified nucleoside comprises a sugar moiety comprising a non-bridging 2'-substituent group selected from: F, NH₂, N₃, OCF₃, OCH₃, O(CH₂)₃NH₂, CH₂CH=CH₂, OCH₂CH=CH₂, OCH₂CH₂OCH₃, O(CH₂)₂SCH₃, O(CH₂)₂ON(Rₘ)(Rₙ), O(CH₂)₂O(CH₂)₂N(CH₃)₂, and N-substituted acetamide (OCH₂C(=O)-N(Rₘ)(Rₙ)), where each Rₙ and Rₙ is, independently, H, an amino protecting group, or substituted or unsubstituted C₁-C₁₀ alkyl.

In certain instances, a 2'-substituted nucleoside non-bicyclic modified nucleoside comprises a sugar moiety comprising a non-bridging 2'-substituent group selected from: F, OCF₃, OCH₃, OCH₂CH₂OCH₃, O(CH₂)₂SCH₃, O(CH₂)₂ON(CH₃)₂, O(CH₂)₂O(CH₂)₂N(CH₃)₂, and OCH₂C(=O)-N(H)CH₃ ("NMA").

In certain instances, a 2'-substituted non-bicyclic modified nucleoside comprises a sugar moiety comprising a non-bridging 2'-substituent group selected from: F, OCH₃, and OCH₂CH₂OCH₃.

Certain modifed sugar moieties comprise a substituent that bridges two atoms of the furanosyl ring to form a second ring, resulting in a bicyclic sugar moiety. In certain such instances, the bicyclic sugar moiety comprises a bridge between the 4' and the 2' furanose ring atoms. Examples of such 4' to 2' bridging sugar substituents include but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt"), 4'-CH₂-O-CH₂-2', 4'-CH₂-N(R)-2', 4'-CH(CH₂OCH₃)-O-2' ("constrained MOE" or "cMOE") and analogs thereof *(see, e.g.,* Seth et al., U.S. 7,399,845, Bhat et al., U.S. 7,569,686, Swayze et al., U.S. 7,741,457, and Swayze et al., U.S. 8,022,193), 4'-C(CH₃)(CH₃)-O-2' and analogs thereof *(see, e.g.,* Seth et al., U.S. 8,278,283), 4'-CH₂-N(OCH₃)-2' and analogs thereof *(see, e.g.,* Prakash et al., U.S. 8,278,425), 4'-CH₂-O-N(CH₃)-2' (*see, e.g.,* Allerson et al., U.S. 7,696,345 and Allerson et al., U.S. 8,124,745), 4'-CH₂-C(H)(CH₃)-2' (*see, e.g.,* Zhou, et al., J. Org. Chem., 2009, 74, 118-134), 4'-CH₂-C(=CH₂)-2' and analogs thereof (*see e.g.,* Seth et al., U.S. 8,278,426), 4'-C(RₐR_{b})-N(R)-O-2', 4'-C(RₐR_{b})-O-N(R)-2', 4'-CH₂-O-N(R)-2', and 4'-CH₂-N(R)-O-2', wherein each R, Rₐ, and R_{b} is, independently, H, a protecting group, or C₁-C₁₂ alkyl *(see, e.g.* Imanishi et al., U.S. 7,427,672).

In certain instances, such 4' to 2' bridges independently comprise from 1 to 4 linked groups independently selected from: -[C(Rₐ)(R_{b})]ₙ-, -[C(Rₐ)(R_{b})]ₙ-O-, -C(Rₐ)=C(R_{b})-, -C(Rₐ)=N-, -C(=NRₐ)-, - C(=O)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Additional bicyclic sugar moieties are known in the art, see, for example: Freier et al., Nucleic Acids Research, 1997, 25(22), 4429-4443, Albaek et al., J. Org. Chem., 2006, 71, 7731-7740, Singh et al., Chem. Commun., 1998, 4, 455-456; Koshkin et al., Tetrahedron, 1998, 54, 3607-3630; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 20017, 129, 8362-8379;Wengel et a., U.S. 7,053,207; Imanishi et al., U.S. 6,268,490; Imanishi et al. U.S. 6,770,748; Imanishi et al., U.S. RE44,779; Wengel et al., U.S. 6,794,499; Wengel et al., U.S. 6,670,461; Wengel et al., U.S. 7,034,133; Wengel et al., U.S. 8,080,644; Wengel et al., U.S. 8,034,909; Wengel et al., U.S. 8,153,365; Wengel et al., U.S. 7,572,582; and Ramasamy et al., U.S. 6,525,191;; Torsten et al., WO 2004/106356;Wengel et al., WO 1999/014226; Seth et al., WO 2007/134181; Seth et al., U.S. 7,547,684; Seth et al., U.S. 7,666,854; Seth et al., U.S. 8,088,746; Seth et al., U.S. 7,750,131; Seth et al., U.S. 8,030,467; Seth et al., U.S. 8,268,980; Seth et al., U.S. 8,546,556; Seth et al., U.S. 8,530,640; Migawa et al., U.S. 9,012,421; Seth et al., U.S. 8,501,805; and U.S. Patent Publication Nos. Allerson et al., US2008/0039618 and Migawa et al., US2015/0191727.

In certain instances, bicyclic sugar moieties and nucleosides incorporating such bicyclic sugar moieties are further defined by isomeric configuration. For example, an LNA nucleoside (described herein) may be in the α-L configuration or in the β-D configuration. 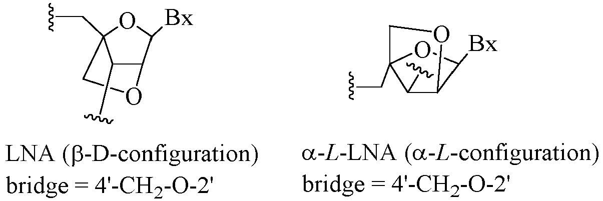 α-L-methyleneoxy (4'-CH₂-O-2') or α-L-LNA bicyclic nucleosides have been incorporated into oligonucleotides that showed antisense activity (Frieden et al., Nucleic Acids Research, 2003, 21, 6365-6372). Herein, general descriptions of bicyclic nucleosides include both isomeric configurations. When the positions of specific bicyclic nucleosides (*e.g*., LNA or cEt) are identified in exemplified instances herein, they are in the β-D configuration, unless otherwise specified.

In certain instances, modified sugar moieties comprise one or more non-bridging sugar substituent and one or more bridging sugar substituent (*e.g.*, 5'-substituted and 4'-2' bridged sugars).

In certain instances, modified sugar moieties are sugar surrogates. In certain such instances, the oxygen atom of the sugar moiety is replaced, *e.g*., with a sulfur, carbon or nitrogen atom. In certain such instances, such modified sugar moieties also comprise bridging and/or non-bridging substituents as described herein. For example, certain sugar surrogates comprise a 4'-sulfur atom and a substitution at the 2'-position *(see, e.g.,* Bhat et al., U.S. 7,875,733 and Bhat et al., U.S. 7,939,677) and/or the 5' position.

In certain instances, sugar surrogates comprise rings having other than 5 atoms. For example, in certain instances, a sugar surrogate comprises a six-membered tetrahydropyran ("THP"). Such tetrahydropyrans may be further modified or substituted. Nucleosides comprising such modified tetrahydropyrans include but are not limited to hexitol nucleic acid ("HNA"), anitol nucleic acid ("ANA"), manitol nucleic acid ("MNA") *(see, e.g.,* Leumann, CJ. Bioorg. & Med. Chem. 2002, 10, 841-854), fluoro HNA:

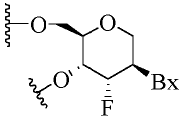

F-HNA

("F-HNA", *see e.g.* Swayze et al., U.S. 8,088,904; Swayze et al., U.S. 8,440,803; Swayze et al., U.S. 8,796,437; and Swayze et al., U.S. 9,005,906; F-HNA can also be referred to as a F-THP or 3'-fluoro tetrahydropyran), and nucleosides comprising additional modified THP compounds having the formula: 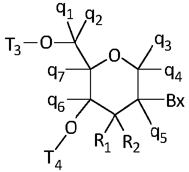 wherein, independently, for each of said modified THP nucleoside:
Bx is a nucleobase moiety;
T₃ and T₄ are each, independently, an internucleoside linking group linking the modified THP nucleoside to the remainder of an oligonucleotide or one of T₃ and T₄ is an internucleoside linking group linking the modified THP nucleoside to the remainder of an oligonucleotide and the other of T₃ and T₄ is H, a hydroxyl protecting group, a linked conjugate group, or a 5' or 3'-terminal group;
   q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from among: hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J₁, J₂, and J₃ is, independently, H or C₁-C₆ alkyl.

In certain instances, modified THP nucleosides are provided wherein q₁, q₂, q₃, q₄, q₅, q₆ and q₇ are each H. In certain instances, at least one of q₁, q₂, q₃, q₄, q₅, q₆ and q₇ is other than H. In certain instances, at least one of q₁, q₂, q₃, q₄, q₅, q₆ and q₇ is methyl. In certain instances, modified THP nucleosides are provided wherein one of R₁ and R₂ is F. In certain instances, R₁ is F and R₂ is H, in certain instances, R₁ is methoxy and R₂ is H, and in certain instances, R₁ is methoxyethoxy and R₂ is H.

In certain instances, sugar surrogates comprise rings having more than 5 atoms and more than one heteroatom. For example, nucleosides comprising morpholino sugar moieties and their use in oligonucleotides have been reported *(see, e.g.,* Braasch et al., Biochemistry, 2002, 41, 4503-4510 and Summerton et al., U.S. 5,698,685; Summerton et al., U.S. 5,166,315; Summerton et al., U.S. 5,185,444; and Summerton et al., U.S. 5,034,506). As used here, the term "morpholino" means a sugar surrogate having the following structure: 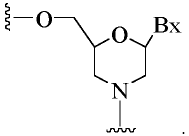 In certain instances, morpholinos may be modified, for example by adding or altering various substituent groups from the above morpholino structure. Such sugar surrogates are refered to herein as "modifed morpholinos."

In certain instances, sugar surrogates comprise acyclic moieites. Examples of nucleosides and oligonucleotides comprising such acyclic sugar surrogates include but are not limited to: peptide nucleic acid ("PNA"), acyclic butyl nucleic acid *(see, e.g.,* Kumar et al., Org. Biomol. Chem., 2013, 11, 5853-5865), and nucleosides and oligonucleotides described in Manoharan et al., WO2011/133876.

Many other bicyclic and tricyclic sugar and sugar surrogate ring systems are known in the art that can be used in modified nucleosides).

### 2. Certain Modified Nucleobases

In certain embodiments, modified oligonucleotides comprise one or more nucleoside comprising an unmodified nucleobase. In certain embodiments, modified oligonucleotides comprise one or more nucleoside comprising a modified nucleobase. In certain instances, modified oligonucleotides comprise one or more nucleoside that does not comprise a nucleobase, referred to as an abasic nucleoside.

In certain embodiments, modified nucleobases are selected from: 5-substituted pyrimidines, 6-azapyrimidines, alkyl or alkynyl substituted pyrimidines, alkyl substituted purines, and N-2, N-6 and O-6 substituted purines. In certain instances, modified nucleobases are selected from: 2-aminopropyladenine, 5-hydroxymethylcytosine, xanthine, hypoxanthine, 2-aminoadenine, 6-N-methylguanine, 6-N-methyladenine, 2-propyladenine , 2-thiouracil, 2-thiothymine and 2-thiocytosine, 5-propynyl (-C≡C-CH₃) uracil, 5-propynylcytosine, 6-azouracil, 6-azocytosine, 6-azothymine, 5-ribosyluracil (pseudouracil), 4-thiouracil, 8-halo, 8-amino, 8-thiol, 8-thioalkyl, 8-hydroxyl, 8-aza and other 8-substituted purines, 5-halo, particularly 5-bromo, 5-trifluoromethyl, 5-halouracil, and 5-halocytosine, 7-methylguanine, 7-methyladenine, 2-F-adenine, 2-aminoadenine, 7-deazaguanine, 7-deazaadenine, 3-deazaguanine, 3-deazaadenine, 6-N-benzoyladenine, 2-N-isobutyrylguanine, 4-N-benzoylcytosine, 4-N-benzoyluracil, 5-methyl 4-N-benzoylcytosine, 5-methyl 4-N-benzoyluracil, universal bases, hydrophobic bases, promiscuous bases, size-expanded bases, and fluorinated bases. Further modified nucleobases include tricyclic pyrimidines, such as 1,3-diazaphenoxazine-2-one, 1,3-diazaphenothiazine-2-one and 9-(2-aminoethoxy)-1,3-diazaphenoxazine-2-one (G-clamp). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in Merigan et al., U.S. 3,687,808, those disclosed in The Concise Encyclopedia Of Polymer Science And Engineering, Kroschwitz, J.I., Ed., John Wiley & Sons, 1990, 858-859; Englisch et al., Angewandte Chemie, International Edition, 1991, 30, 613; Sanghvi, Y.S., Chapter 15, Antisense Research and Applications, Crooke, S.T. and Lebleu, B., Eds., CRC Press, 1993, 273-288; and those disclosed in Chapters 6 and 15, Antisense Drug Technology, Crooke S.T., Ed., CRC Press, 2008, 163-166 and 442-443.

Publications that teach the preparation of certain of the above noted modified nucleobases as well as other modified nucleobases include without limitation, Manohara et al., US2003/0158403; Manoharan et al., US2003/0175906; Dinh et al., U.S. 4,845,205; Spielvogel et al., U.S. 5,130,302; Rogers et al., U.S. 5,134,066; Bischofberger et al., U.S. 5,175,273; Urdea et al., U.S. 5,367,066; Benner et al., U.S. 5,432,272; Matteucci et al., U.S. 5,434,257; Gmeiner et al., U.S. 5,457,187; Cook et al., U.S. 5,459,255; Froehler et al., U.S. 5,484,908; Matteucci et al., U.S. 5,502,177; Hawkins et al., U.S. 5,525,711; Haralambidis et al., U.S. 5,552,540; Cook et al., U.S. 5,587,469; Froehler et al., U.S. 5,594,121; Switzer et al., U.S. 5,596,091; Cook et al., U.S. 5,614,617; Froehler et al., U.S. 5,645,985; Cook et al., U.S. 5,681,941; Cook et al., U.S. 5,811,534; Cook et al., U.S. 5,750,692; Cook et al., U.S. 5,948,903; Cook et al., U.S. 5,587,470; Cook et al., U.S. 5,457,191; Matteucci et al., U.S. 5,763,588; Froehler et al., U.S. 5,830,653; Cook et al., U.S. 5,808,027; Cook et al., 6,166,199; and Matteucci et al., U.S. 6,005,096.

### 3. Certain Modified Internucleoside Linkages

Nucleosides of modified oligonucleotides may be linked together using any internucleoside linkage. The two main classes of internucleoside linking groups are defined by the presence or absence of a phosphorus atom. Representative phosphorus-containing internucleoside linkages include but are not limited to phosphates, which contain a phosphodiester bond ("P=O") (also referred to as unmodified or naturally occurring linkages), phosphotriesters, methylphosphonates, phosphoramidates, and phosphorothioates ("P=S"), and phosphorodithioates ("HS-P=S"). Representative non-phosphorus containing internucleoside linking groups include but are not limited to methylenemethylimino (-CH₂-N(CH₃)-O-CH₂-), thiodiester, thionocarbamate (-O-C(=O)(NH)-S-); siloxane (-O-SiH₂-O-); and N,N'-dimethylhydrazine (-CH₂-N(CH₃)-N(CH₃)-). Modified internucleoside linkages, compared to naturally occurring phosphate linkages, can be used to alter, typically increase, nuclease resistance of the oligonucleotide. Methods of preparation of phosphorous-containing and non-phosphorous-containing internucleoside linkages are well known to those skilled in the art.

Representative internucleoside linkages having a chiral center include but are not limited to alkylphosphonates and phosphorothioates. Modified oligonucleotides comprising internucleoside linkages having a chiral center can be prepared as populations of modified oligonucleotides comprising stereorandom internucleoside linkages, or as populations of modified oligonucleotides comprising phosphorothioate linkages in particular stereochemical configurations. In certain embodiments, populations of modified oligonucleotides comprise phosphorothioate internucleoside linkages wherein all of the phosphorothioate internucleoside linkages are stereorandom. Such modified oligonucleotides can be generated using synthetic methods that result in random selection of the stereochemical configuration of each phosphorothioate linkage. Nonetheless, as is well understood by those of skill in the art, each individual phosphorothioate of each individual oligonucleotide molecule has a defined stereoconfiguration. In certain embodiments, populations of modified oligonucleotides are enriched for modified oligonucleotides comprising one or more particular phosphorothioate internucleoside linkages in a particular, independently selected stereochemical configuration. In certain embodiments, the particular configuration of the particular phosphorothioate linkage is present in at least 65% of the molecules in the population. In certain embodiments, the particular configuration of the particular phosphorothioate linkage is present in at least 70% of the molecules in the population. In certain embodiments, the particular configuration of the particular phosphorothioate linkage is present in at least 80% of the molecules in the population. In certain embodiments, the particular configuration of the particular phosphorothioate linkage is present in at least 90% of the molecules in the population. In certain embodiments, the particular configuration of the particular phosphorothioate linkage is present in at least 99% of the molecules in the population. Such chirally enriched populations of modified oligonucleotides can be generated using synthetic methods known in the art, *e.g*., methods described in Oka et al., JACS 125, 8307 (2003), Wan et al. Nuc. Acid. Res. 42, 13456 (2014), and WO 2017/015555. In certain embodiments, a population of modified oligonucleotides is enriched for modified oligonucleotides having at least one indicated phosphorothioate in the (Sp) configuration. In certain embodiments, a population of modified oligonucleotides is enriched for modified oligonucleotides having at least one phosphorothioate in the (Rp) configuration. In certain embodiments, modified oligonucleotides comprising (Rp) and/or (Sp) phosphorothioates comprise one or more of the following formulas, respectively, wherein "B" indicates a nucleobase: 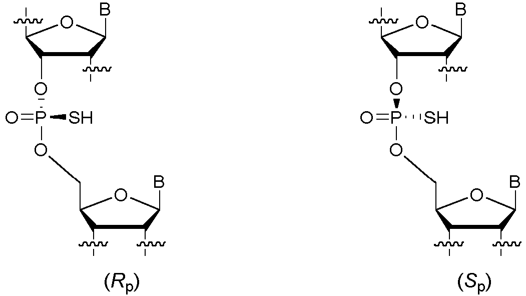 Unless otherwise indicated, chiral internucleoside linkages of modified oligonucleotides described herein can be stereorandom or in a particular stereochemical configuration.

Neutral internucleoside linkages include, without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), methoxypropyl, and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### B. Certain Motifs

In certain embodiments, modified oligonucleotides comprise one or more modified nucleosides comprising a modified sugar moiety. In certain embodiments, modified oligonucleotides comprise one or more modified nucleosides comprising a modified nucleobase. In certain embodiments, modified oligonucleotides comprise one or more modified internucleoside linkage. In such embodiments, the modified, unmodified, and differently modified sugar moieties, nucleobases, and/or internucleoside linkages of a modified oligonucleotide define a pattern or motif. In certain embodiments, the patterns of sugar moieties, nucleobases, and internucleoside linkages are each independent of one another. Thus, a modified oligonucleotide may be described by its sugar motif, nucleobase motif and/or internucleoside linkage motif (as used herein, nucleobase motif describes the modifications to the nucleobases independent of the sequence of nucleobases).

### 1. Certain Sugar Motifs

In certain embodiments, oligonucleotides comprise one or more type of modified sugar and/or unmodified sugar moiety arranged along the oligonucleotide or region thereof in a defined pattern or sugar motif. In certain instances, such sugar motifs include but are not limited to any of the sugar modifications discussed herein.

In certain embodiments, modified oligonucleotides comprise or consist of a region having a gapmer motif, which is defined by two external regions or "wings" and a central or internal region or "gap." The three regions of a gapmer motif (the 5'-wing, the gap, and the 3'-wing) form a contiguous sequence of nucleosides wherein at least some of the sugar moieties of the nucleosides of each of the wings differ from at least some of the sugar moieties of the nucleosides of the gap. Specifically, at least the sugar moieties of the nucleosides of each wing that are closest to the gap (the 3'-most nucleoside of the 5'-wing and the 5'-most nucleoside of the 3'-wing) differ from the sugar moiety of the neighboring gap nucleosides, thus defining the boundary between the wings and the gap (i.e., the wing/gap junction). In certain embodiments, the sugar moieties within the gap are the same as one another. In certain instances, the gap includes one or more nucleoside having a sugar moiety that differs from the sugar moiety of one or more other nucleosides of the gap. In certain embodiments, the sugar motifs of the two wings are the same as one another (symmetric gapmer). In certain instances, the sugar motif of the 5'-wing differs from the sugar motif of the 3'-wing (asymmetric gapmer).

In certain embodiments, each nucleoside of each wing of a gapmer is a modified nucleoside.

In certain embodiments, each nucleoside of the gap of a gapmer is an unmodified 2'-deoxy nucleoside.

In certain embodiments, the gapmer is a deoxy gapmer. In embodiments, the nucleosides on the gap side of each wing/gap junction are unmodified 2'-deoxy nucleosides and the nucleosides on the wing sides of each wing/gap junction are modified nucleosides. In certain embodiments, each nucleoside of the gap is an unmodified 2'-deoxy nucleoside. In certain embodiments, each nucleoside of each wing of a gapmer is a modified nucleoside.

In certain embodiments, modified oligonucleotides comprise or consist of a region having a fully modified sugar motif. In such embodiments, each nucleoside of the fully modified region of the modified oligonucleotide comprises a modified sugar moiety. In certain instances, each nucleoside of the entire modified oligonucleotide comprises a modified sugar moiety. In certain embodiments, modified oligonucleotides comprise or consist of a region having a fully modified sugar motif, wherein each nucleoside within the fully modified region comprises the same modified sugar moiety, referred to herein as a uniformly modified sugar motif. In certain instances, a fully modified oligonucleotide is a uniformly modified oligonucleotide. In certain instances, each nucleoside of a uniformly modified comprises the same 2'-modification.

Herein, the lengths (number of nucleosides) of the three regions of a gapmer may be provided using the notation [# of nucleosides in the 5'-wing] - [# of nucleosides in the gap] - [# of nucleosides in the 3'-wing]. Thus, a 5-8-5 gapmer consists of 5 linked nucleosides in each wing and 8 linked nucleosides in the gap. Where such nomenclature is followed by a specific modification, that modification is the modification in the wings and the gap nucleosides comprise unmodified deoxynucleosides sugars. Thus, a 5-8-5 MOE gapmer consists of 5 linked MOE modified nucleosides in the 5'-wing, 8 linked deoxynucleosides in the gap, and 5 linked MOE nucleosides in the 3'-wing. In certain embodiments, modified oligonucleotides are 5-8-5 MOE gapmers.

### 2. Certain Nucleobase Motifs

In certain embodiments, oligonucleotides comprise modified and/or unmodified nucleobases arranged along the oligonucleotide or region thereof in a defined pattern or motif. In certain instances, each nucleobase is modified. In certain instances, none of the nucleobases are modified. In certain instances, each purine or each pyrimidine is modified. In certain instances, each adenine is modified. In certain instances, each guanine is modified. In certain instances, each thymine is modified. In certain instances, each uracil is modified. In certain embodiments, each cytosine is modified. In certain embodiments, some or all of the cytosine nucleobases in a modified oligonucleotide are 5-methylcytosines. In certain embodiments, all of the cytosine nucleobases are 5-methylcytosines and all of the other nucleobases of the modified oligonucleotide are unmodified nucleobases.

In certain embodiments, modified oligonucleotides comprise a block of modified nucleobases. In certain such embodiments, the block is at the 3'-end of the oligonucleotide. In certain embodiments the block is within 3 nucleosides of the 3'-end of the oligonucleotide. In certain embodiments, the block is at the 5'-end of the oligonucleotide. In certain embodiments the block is within 3 nucleosides of the 5'-end of the oligonucleotide.

In certain embodiments, oligonucleotides having a gapmer motif comprise a nucleoside comprising a modified nucleobase. In certain such embodiments, one nucleoside comprising a modified nucleobase is in the central gap of an oligonucleotide having a gapmer motif. In certain such embodiments, the sugar moiety of said nucleoside is a 2'-deoxyribosyl moiety. In certain instances, the modified nucleobase is selected from: a 2-thiopyrimidine and a 5-propynepyrimidine.

### 3. Certain Internucleoside Linkage Motifs

In certain embodiments, oligonucleotides comprise modified and/or unmodified internucleoside linkages arranged along the oligonucleotide or region thereof in a defined pattern or motif. In certain instances, each internucleoside linking group is a phosphodiester internucleoside linkage (P=O). In certain instances, each internucleoside linking group of a modified oligonucleotide is a phosphorothioate internucleoside linkage (P=S). In certain embodiments, each internucleoside linkage of a modified oligonucleotide is independently selected from a phosphorothioate internucleoside linkage and phosphodiester internucleoside linkage. In certain embodiments, each phosphorothioate internucleoside linkage is independently selected from a stereorandom phosphorothioate, a (Sp) phosphorothioate, and a (*Rp*) phosphorothioate. In certain embodiments, the sugar motif of a modified oligonucleotide is a gapmer and the internucleoside linkages within the gap are all modified. In certain such embodiments, some of the internucleoside linkages in the wings are unmodified phosphate linkages. In certain embodiments, the terminal internucleoside linkages are modified. In certain embodiments, the sugar motif of a modified oligonucleotide is a gapmer, and the internucleoside linkage motif comprises at least one phosphodiester internucleoside linkage in at least one wing, wherein the at least one phosphodiester linkage is not a terminal internucleoside linkage, and the remaining internucleoside linkages are phosphorothioate internucleoside linkages. In certain such embodiments, all of the phosphorothioate linkages are stereorandom. In certain embodiments, all of the phosphorothioate linkages in the wings are (Sp) phosphorothioates, and the gap comprises at least one *Sp, Sp, Rp* motif. In certain embodiments, populations of modified oligonucleotides are enriched for modified oligonucleotides comprising such internucleoside linkage motifs.

### C. Certain Lengths

It is possible to increase or decrease the length of an oligonuclotide without eliminating activity. For example, in Woolf et al. (Proc. Natl. Acad. Sci. USA 89:7305-7309, 1992), a series of oligonucleotides 13-25 nucleobases in length were tested for their ability to induce cleavage of a target RNA in an oocyte injection model. Oligonucleotides 25 nucleobases in length with 8 or 11 mismatch bases near the ends of the oligonucleotides were able to direct specific cleavage of the target mRNA, albeit to a lesser extent than the oligonucleotides that contained no mismatches. Similarly, target specific cleavage was achieved using 13 nucleobase oligonucleotides, including those with 1 or 3 mismatches.

The modified oligonucleotides for use of the invention have a length of 18 linked nucleosides.

### D. Certain Modified Oligonucleotides

In certain embodiments, the above modifications (sugar, nucleobase, internucleoside linkage) are incorporated into a modified oligonucleotide. In certain embodiments, modified oligonucleotides are characterized by their modification motifs and overall lengths. In certain embodiments, such parameters are each independent of one another. Thus, unless otherwise indicated, each internucleoside linkage of an oligonucleotide having a gapmer sugar motif may be modified or unmodified and may or may not follow the gapmer modification pattern of the sugar modifications. For example, the internucleoside linkages within the wing regions of a sugar gapmer may be the same or different from one another and may be the same or different from the internucleoside linkages of the gap region of the sugar motif. Likewise, such sugar gapmer oligonucleotides may comprise one or more modified nucleobase independent of the gapmer pattern of the sugar modifications. Unless otherwise indicated, all modifications are independent of nucleobase sequence.

### E. Certain populations of modified oligonucleotides

Populations of modified oligonucleotides in which all of the modified oligonucleotides of the population have the same molecular formula can be stereorandom populations or chirally enriched populations. All of the chiral centers of all of the modified oligonucleotides are stereorandom in a stereorandom population. In a chirally enriched population, at least one particular chiral center is not stereorandom in the modified oligonucleotides of the population. In certain embodiments, the modified oligonucleotides of a chirally enriched population are enriched for β-D ribosyl sugar moieties, and all of the phosphorothioate internucleoside linkages are stereorandom. In certain embodiments, the modified oligonucltoides of a chirally enriched population are enriched for both β-D ribosyl sugar moieties and at least one, particular phosphorothioate internucleoside linkage in a particular sterochemical configuration.

### F. Nucleobase Sequence

In certain embodiments, oligonucleotides (unmodified or modified oligonucleotides) are further described by their nucleobase sequence. In certain embodiments oligonucleotides have a nucleobase sequence that is complementary to a second oligonucleotide or an identified reference nucleic acid, such as a target nucleic acid. In certain such embodiments, a region of an oligonucleotide has a nucleobase sequence that is complementary to a second oligonucleotide or an identified reference nucleic acid, such as a target nucleic acid. In certain embodiments, the nucleobase sequence of a region or entire length of an oligonucleotide is at least 50%, at least 60%, at least 70%, at least 80%, at least 85%, at least 90%, at least 95%, or 100% complementary to the second oligonucleotide or nucleic acid, such as a target nucleic acid.

### II. Certain Oligomeric Compounds

In certain embodiments, the invention provides oligomeric compounds, which consist of an oligonucleotide (modified or unmodified) and optionally one or more conjugate groups and/or terminal groups, as defined in the claims. Conjugate groups consist of one or more conjugate moiety and a conjugate linker which links the conjugate moiety to the oligonucleotide. Conjugate groups may be attached to either or both ends of an oligonucleotide and/or at any internal position. In certain embodiments, conjugate groups are attached to the 2'-position of a nucleoside of a modified oligonucleotide. In certain embodiments, conjugate groups that are attached to either or both ends of an oligonucleotide are terminal groups. In certain such embodiments, conjugate groups or terminal groups are attached at the 3' and/or 5'-end of oligonucleotides. In certain such embodiments, conjugate groups (or terminal groups) are attached at the 3'-end of oligonucleotides. In certain embodiments, conjugate groups are attached near the 3'-end of oligonucleotides. In certain embodiments, conjugate groups (or terminal groups) are attached at the 5'-end of oligonucleotides. In certain embodiments, conjugate groups are attached near the 5'-end of oligonucleotides.

Examples of terminal groups include but are not limited to conjugate groups, capping groups, phosphate moieties, protecting groups, modified or unmodified nucleosides, and two or more nucleosides that are independently modified or unmodified.

### A. Certain Conjugate Groups

In certain embodiments, oligonucleotides are covalently attached to one or more conjugate groups. In certain embodiments, conjugate groups modify one or more properties of the attached oligonucleotide, including but not limited to pharmacodynamics, pharmacokinetics, stability, binding, absorption, tissue distribution, cellular distribution, cellular uptake, charge and clearance. In certain embodiments, conjugate groups impart a new property on the attached oligonucleotide, *e.g.*, fluorophores or reporter groups that enable detection of the oligonucleotide. Certain conjugate groups and conjugate moieties have been described previously, for example: cholesterol moiety (Letsinger et al., Proc. Natl. Acad. Sci. USA, 1989, 86, 6553-6556), cholic acid (Manoharan et al., Bioorg. Med. Chem. Lett., 1994, 4, 1053-1060), a thioether, *e.g.,* hexyl-S-tritylthiol (Manoharan et al., Ann. N.Y. Acad. Sci., 1992, 660, 306-309; Manoharan et al., Bioorg. Med. Chem. Lett., 1993, 3, 2765-2770), a thiocholesterol (Oberhauser et al., Nucl. Acids Res., 1992, 20, 533-538), an aliphatic chain, *e.g.,* do-decan-diol or undecyl residues (Saison-Behmoaras et al., EMBO J, 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid a palmityl moiety (Mishra et al., Biochim. Biophys. Acta, 1995, 1264, 229-237), an octadecylamine or hexylamino-carbonyl-oxycholesterol moiety (Crooke et al., J. Pharmacol. Exp. Ther., 1996, 277, 923-937), a tocopherol group (Nishina et al., Molecular Therapy Nucleic Acids, 2015, 4, e220; and Nishina et al., Molecular Therapy, 2008, 16, 734-740), or a GalNAc cluster (*e.g*., WO2014/179620).

### 1. Conjugate Moieties

Conjugate moieties include, without limitation, intercalators, reporter molecules, polyamines, polyamides, peptides, carbohydrates, vitamin moieties, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins, fluorophores, and dyes.

In certain embodiments, a conjugate moiety comprises an active drug substance, for example, aspirin, warfarin, phenylbutazone, ibuprofen, suprofen, fen-bufen, ketoprofen, (*S*)-(+)-pranoprofen, carprofen, dansylsarcosine, 2,3,5-triiodobenzoic acid, fingolimod, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

### 2. Conjugate Linkers

Conjugate moieties are attached to oligonucleotides through conjugate linkers. In certain oligomeric compounds, the conjugate linker is a single chemical bond (i.e., the conjugate moiety is attached directly to an oligonucleotide through a single bond). In certain embodiments, the conjugate linker comprises a chain structure, such as a hydrocarbyl chain, or an oligomer of repeating units such as ethylene glycol, nucleosides, or amino acid units.

In certain embodiments, a conjugate linker comprises one or more groups selected from alkyl, amino, oxo, amide, disulfide, polyethylene glycol, ether, thioether, and hydroxylamino. In certain such embodiments, the conjugate linker comprises groups selected from alkyl, amino, oxo, amide and ether groups. In certain embodiments, the conjugate linker comprises groups selected from alkyl and amide groups. In certain embodiments, the conjugate linker comprises groups selected from alkyl and ether groups. In certain embodiments, the conjugate linker comprises at least one phosphorus moiety. In certain embodiments, the conjugate linker comprises at least one phosphate group. In certain embodiments, the conjugate linker includes at least one neutral linking group.

In certain embodiments, conjugate linkers, including the conjugate linkers described above, are bifunctional linking moieties, *e.g*., those known in the art to be useful for attaching conjugate groups to parent compounds, such as the oligonucleotides provided herein. In general, a bifunctional linking moiety comprises at least two functional groups. One of the functional groups is selected to bind to a particular site on a parent compound and the other is selected to bind to a conjugate group. Examples of functional groups used in a bifunctional linking moiety include but are not limited to electrophiles for reacting with nucleophilic groups and nucleophiles for reacting with electrophilic groups. In certain embodiments, bifunctional linking moieties comprise one or more groups selected from amino, hydroxyl, carboxylic acid, thiol, alkyl, alkenyl, and alkynyl.

Examples of conjugate linkers include but are not limited to pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other conjugate linkers include but are not limited to substituted or unsubstituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

In certain embodiments, conjugate linkers comprise 1-10 linker-nucleosides. In certain embodiments, conjugate linkers comprise 2-5 linker-nucleosides. In certain embodiments, conjugate linkers comprise exactly 3 linker-nucleosides. In certain embodiments, conjugate linkers comprise the TCA motif. In certain embodiments, such linker-nucleosides are modified nucleosides. In certain embodiments such linker-nucleosides comprise a modified sugar moiety. In certain embodiments, linker-nucleosides are unmodified. In certain embodiments, linker-nucleosides comprise an optionally protected heterocyclic base selected from a purine, substituted purine, pyrimidine or substituted pyrimidine. In certain embodiments, a cleavable moiety is a nucleoside selected from uracil, thymine, cytosine, 4-N-benzoylcytosine, 5-methylcytosine, 4-N-benzoyl-5-methylcytosine, adenine, 6-N-benzoyladenine, guanine and 2-N-isobutyrylguanine. It is typically desirable for linker-nucleosides to be cleaved from the oligomeric compound after it reaches a target tissue. Accordingly, linker-nucleosides are typically linked to one another and to the remainder of the oligomeric compound through cleavable bonds. In certain embodimements, such cleavable bonds are phosphodiester bonds.

Herein, linker-nucleosides are not considered to be part of the oligonucleotide. Accordingly, in embodiments in which an oligomeric compound comprises an oligonucleotide consisting of a specified number or range of linked nucleosides and/or a specified percent complementarity to a reference nucleic acid and the oligomeric compound also comprises a conjugate group comprising a conjugate linker comprising linker-nucleosides, those linker-nucleosides are not counted toward the length of the oligonucleotide and are not used in determining the percent complementarity of the oligonucleotide for the reference nucleic acid. For example, an oligomeric compound may comprise (1) a modified oligonucleotide consisting of 8-30 nucleosides and (2) a conjugate group comprising 1-10 linker-nucleosides that are contiguous with the nucleosides of the modified oligonucleotide. The total number of contiguous linked nucleosides in such an oligomeric compound is more than 30. Alternatively, an oligomeric compound may comprise a modified oligonucleotide consisting of 8-30 nucleosides and no conjugate group. The total number of contiguous linked nucleosides in such an oligomeric compound is no more than 30. Unless otherwise indicated conjugate linkers comprise no more than 10 linker-nucleosides. In certain embodiments, conjugate linkers comprise no more than 5 linker-nucleosides. In certain embodiments, conjugate linkers comprise no more than 3 linker-nucleosides. In certain embodiments, conjugate linkers comprise no more than 2 linker-nucleosides. In certain embodiments, conjugate linkers comprise no more than 1 linker-nucleoside.

In certain embodiments, it is desirable for a conjugate group to be cleaved from the oligonucleotide. For example, in certain circumstances oligomeric compounds comprising a particular conjugate moiety are better taken up by a particular cell type, but once the oligomeric compound has been taken up, it is desirable that the conjugate group be cleaved to release the unconjugated or parent oligonucleotide. Thus, certain conjugate linkers may comprise one or more cleavable moieties. In certain embodiments, a cleavable moiety is a cleavable bond. In certain embodiments, a cleavable moiety is a group of atoms comprising at least one cleavable bond. In certain embodiments, a cleavable moiety comprises a group of atoms having one, two, three, four, or more than four cleavable bonds. In certain embodiments, a cleavable moiety is selectively cleaved inside a cell or subcellular compartment, such as a lysosome. In certain embodiments, a cleavable moiety is selectively cleaved by endogenous enzymes, such as nucleases.

In certain embodiments, a cleavable bond is selected from among: an amide, an ester, an ether, one or both esters of a phosphodiester, a phosphate ester, a carbamate, or a disulfide. In certain embodiments, a cleavable bond is one or both of the esters of a phosphodiester. In certain embodiments, a cleavable moiety comprises a phosphate or phosphodiester. In certain embodiments, the cleavable moiety is a phosphate linkage between an oligonucleotide and a conjugate moiety or conjugate group.

In certain embodiments, a cleavable moiety comprises or consists of one or more linker-nucleosides. In certain such embodiments, the one or more linker-nucleosides are linked to one another and/or to the remainder of the oligomeric compound through cleavable bonds. In certain embodiments, such cleavable bonds are unmodified phosphodiester bonds. In certain embodiments, a cleavable moiety is 2'-deoxy nucleoside that is attached to either the 3' or 5'-terminal nucleoside of an oligonucleotide by a phosphate internucleoside linkage and covalently attached to the remainder of the conjugate linker or conjugate moiety by a phosphate or phosphorothioate linkage. In certain such embodiments, the cleavable moiety is 2'-deoxyadenosine.

### B. Certain Terminal Groups

In certain embodiments, oligomeric compounds comprise one or more terminal groups. In certain such embodiments, oligomeric compounds comprise a stabilized 5'-phophate. Stabilized 5'-phosphates include, but are not limited to 5'-phosphanates, including, but not limited to 5'-vinylphosphonates. In certain embodiments, terminal groups comprise one or more abasic nucleosides and/or inverted nucleosides. In certain embodiments, terminal groups comprise one or more 2'-linked nucleosides. In certain such embodiments, the 2'-linked nucleoside is an abasic nucleoside.

### III. Oligomeric Duplexes

In certain embodiments, oligomeric compounds described herein comprise an oligonucleotide, having a nucleobase sequence complementary to that of a target nucleic acid. In certain embodiments, an oligomeric compound is paired with a second oligomeric compound to form an oligomeric duplex. Such oligomeric duplexes comprise a first oligomeric compound having a region complementary to a target nucleic acid and a second oligomeric compound having a region complementary to the first oligomeric compound. In certain embodiments, the first oligomeric compound of an oligomeric duplex comprises or consists of (1) a modified or unmodified oligonucleotide and optionally a conjugate group and (2) a second modified or unmodified oligonucleotide and optionally a conjugate group. Either or both oligomeric compounds of an oligomeric duplex may comprise a conjugate group. The oligonucleotides of each oligomeric compound of an oligomeric duplex may include non-complementary overhanging nucleosides.

### IV. Antisense Activity

In certain embodiments, oligomeric compounds and oligomeric duplexes are capable of hybridizing to a target nucleic acid, resulting in at least one antisense activity; such oligomeric compounds and oligomeric duplexes are antisense compounds. In certain embodiments, antisense compounds have antisense activity when they reduce or inhibit the amount or activity of a target nucleic acid by 25% or more in the standard cell assay. In certain embodiments, antisense compounds selectively affect one or more target nucleic acid. Such antisense compounds comprise a nucleobase sequence that hybridizes to one or more target nucleic acid, resulting in one or more desired antisense activity and does not byridize to one or more non-target nucleic acid or does not hybridize to one or more non-target nucleic acid in such a way that results in significant undesired antisense activity.

In certain antisense activities, hybridization of an antisense compound to a target nucleic acid results in recruitment of a protein that cleaves the target nucleic acid. For example, certain antisense compounds result in RNase H mediated cleavage of the target nucleic acid. RNase H is a cellular endonuclease that cleaves the RNA strand of an RNA:DNA duplex. The DNA in such an RNA:DNA duplex need not be unmodified DNA. In certain embodiments, described herein are antisense compounds that are sufficiently "DNA-like" to elicit RNase H activity. In certain embodiments, one or more non-DNA-like nucleoside in the gap of a gapmer is tolerated.

In certain antisense activities, an antisense compound or a portion of an antisense compound is loaded into an RNA-induced silencing complex (RISC), ultimately resulting in cleavage of the target nucleic acid. For example, certain antisense compounds result in cleavage of the target nucleic acid by Argonaute. Antisense compounds that are loaded into RISC are RNAi compounds. RNAi compounds may be double-stranded (siRNA) or single-stranded (ssRNA).

In certain embodiments, hybridization of an antisense compound to a target nucleic acid does not result in recruitment of a protein that cleaves that target nucleic acid. In certain embodiments, hybridization of the antisense compound to the target nucleic acid results in alteration of splicing of the target nucleic acid. In certain embodiments, hybridization of an antisense compound to a target nucleic acid results in inhibition of a binding interaction between the target nucleic acid and a protein or other nucleic acid. In certain embodiments, hybridization of an antisense compound to a target nucleic acid results in alteration of translation of the target nucleic acid.

Antisense activities may be observed directly or indirectly. In certain embodiments, observation or detection of an antisense activity involves observation or detection of a change in an amount of a target nucleic acid or protein encoded by such target nucleic acid, a change in the ratio of splice variants of a nucleic acid or protein, and/or a phenotypic change in a cell or animal.

### V. Certain Target Nucleic Acids

In certain embodiments, oligomeric compounds comprise or consist of an oligonucleotide comprising a region that is complementary to a target nucleic acid. In certain embodiments, the target nucleic acid is an endogenous RNA molecule. In certain embodiments, the target nucleic acid encodes a protein. In certain such embodiments, the target nucleic acid is selected from: a mature mRNA and a pre-mRNA, including intronic, exonic and untranslated regions. In certain embodiments, the target RNA is a mature mRNA. In certain embodiments, the target nucleic acid is a pre-mRNA. In certain such embodiments, the target region is entirely within an intron. In certain embodiments, the target region spans an intron/exon junction. In certain embodiments, the target region is at least 50% within an intron.

### A. Complementarity/Mismatches to the Target Nucleic Acid

It is possible to incroduce mismatch bases without eliminating activity. For example, Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL in vitro and in vivo. Furthermore, this oligonucleotide demonstrated potent antitumor activity in vivo. Maher and Dolnick (Nuc. Acid. Res. 16:3341-3358, 1988) tested a series of tandem 14 nucleobase oligonucleotides, and a 28 and 42 nucleobase oligonucleotides comprised of the sequence of two or three of the tandem oligonucleotides, respectively, for their ability to arrest translation of human DHFR in a rabbit reticulocyte assay. Each of the three 14 nucleobase oligonucleotides alone was able to inhibit translation, albeit at a more modest level than the 28 or 42 nucleobase oligonucleotides.

In certain embodiments, oligomeric compounds comprise oligonucletoides that are complementary to the target nucleic acid over the entire length of the oligonucleotide. In certain embodiments, oligonucleotides are 99%, 95%, 90%, 85%, or 80% complementary to the target nucleic acid. In certain embodiments, oligonucleotides are at least 80% complementary to the target nucleic acid over the entire length of the oligonucleotide and comprise a region that is 100% or fully complementary to a target nucleic acid. In certain embodiments, the region of full complementarity is from 6 to 20, 10 to 18, or 18 to 20 nucleobases in length.

In certain embodiments, oligonucleotides comprise one or more mismatched nucleobases relative to the target nucleic acid. In certain embodiments, antisense activity against the target is reduced by such mismatch, but activity against a non-target is reduced by a greater amount. Thus, in certain embodiments selectivity of the oligomeric compound comprising an oligonucleotide is improved. In certain embodiments, the mismatch is specifically positioned within an oligonucleotide having a gapmer motif. In certain embodiments, the mismatch is at position 1, 2, 3, 4, 5, 6, 7, or 8 from the 5'-end of the gap region. In certain embodiments, the mismatch is at position 9, 8, 7, 6, 5, 4, 3, 2, 1 from the 3'-end of the gap region. In certain embodiments, the mismatch is at position 1, 2, 3, or 4 from the 5'-end of the wing region. In certain embodiments, the mismatch is at position 4, 3, 2, or 1 from the 3'-end of the wing region.

### B. Tau

The oligomeric compounds for use of the invention comprise or consist of an oligonucleotide comprising a region that is complementary to a target nucleic acid, wherein the target nucleic acid is Tau. In certain embodiments, Tau nucleic acid has the sequence set forth in SEQ ID NO: 1 (GENBANK Accession No. NT_010783.14 truncated from nucleotides 2624000 to 2761000).

In certain embodiments, contacting a cell with an oligomeric compound complementary to SEQ ID NO: 1 reduces the amount of Tau mRNA, and in certain embodiments reduces the amount of Tau protein. In certain embodiments, contacting a cell in ahuman subject with an oligomeric compound complementary to SEQ ID NO: 1 ameliroates one or more symptoms of a neurodegenerative disease. In certain embodiments, the symptom is loss of memory, loss of motor function, or increase in the number and/or volume of neurofibrillary inclusions. In certain embodiments, contacting a cell in a human subject with an oligonucleotide complementary to SEQ ID NO: 1 results in maintining or improving memory, maintaining or improving motor function, and/or maintenance or reduction in the number and/or volume of neurofibrillary inclusions.

### C. Certain Target Nucleic Acids in Certain Tissues

In certain embodiments, oligomeric compounds comprise or consist of an oligonucleotide comprising a region that is complementary to a target nucleic acid, wherein the target nucleic acid is expressed in the central nervous system (CNS).

### VI. Certain Pharmaceutical Compositions

In certain embodiments, described herein are pharmaceutical compositions comprising one or more oligomeric compounds or a salt thereof, in accordance with the claims. In certain embodiments, the pharmaceutical composition comprises a pharmaceutically acceptable diluent or carrier. In certain embodiments, a pharmaceutical composition comprises a sterile saline solution and one or more oligomeric compound. In certain embodiments, a pharmaceutical composition consists of a sterile saline solution and one or more oligomeric compound. In certain embodiments, the sterile saline is pharmaceutical grade saline. In certain embodiments, a pharmaceutical composition comprises one or more oligomeric compound and sterile water. In certain embodiments, a pharmaceutical composition consists of one oligomeric compound and sterile water. In certain embodiments, the sterile water is pharmaceutical grade water. In certain embodiments, a pharmaceutical composition comprises one or more oligomeric compound and phosphate-buffered saline (PBS). In certain embodiments, a pharmaceutical composition consists of one or moreoligomeric compound and sterile PBS. In certain embodiments, the sterile PBS is pharmaceutical grade PBS.

In certain embodiments, pharmaceutical compositions comprise one or more oligomeric compound and one or more excipients. In certain embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, oligomeric compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

In certain embodiments, pharmaceutical compositions comprising an oligomeric compound encompass any pharmaceutically acceptable salts of the oligomeric compound, esters of the oligomeric compound, or salts of such esters. In certain embodiments, pharmaceutical compositions comprising oligomeric compounds comprising one or more oligonucleotide, upon administration to an animal, including a human, are capable of providing (directly or indirectly) the biologically active metabolite or residue thereof. Accordingly, for example, the disclosure is also drawn to pharmaceutically acceptable salts of oligomeric compounds, prodrugs, pharmaceutically acceptable salts of such prodrugs, and other bioequivalents. Suitable pharmaceutically acceptable salts include, but are not limited to, sodium and potassium salts. In certain embodiments, prodrugs comprise one or more conjugate group attached to an oligonucleotide, wherein the conjugate group is cleaved by endogenous nucleases within the body.

Lipid moieties have been used in nucleic acid therapies in a variety of methods. In certain such methods, the nucleic acid, such as an oligomeric compound, is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions comprise a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, pharmaceutical compositions comprise one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, pharmaceutical compositions comprise a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80^{™} and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80^{™}; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, pharmaceutical compositions are prepared for oral administration. In certain embodiments, pharmaceutical compositions are prepared for buccal administration. In certain embodiments, a pharmaceutical composition is prepared for administration by injection (e.g., intravenous, subcutaneous, intramuscular, intrathecal, intracerebroventricular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain.

### VII. Certain Compounds

In certain embodiments, Compound No. 814907 is characterized as a 5-8-5 MOE gapmer, having a sequence of (from 5' to 3') CCGTTTTCTTACCACCCT (SEQ ID NO: 8), wherein each of nucleosides 1-5 and 14-18 are 2'-MOE nucleosides and each of nucleosides 6-13 are 2'-deoxynucleosides, wherein the internucleoside linkages between nucleoside 2 and nucleoside 3 and nucleoside 15 to nucleoside 16 are phosphodiester internucleoside linkages and the remainder of the internucleoside linkages are phosphorothioate internucleoside linkages, and wherein each cytosine is a 5-methylcytosine.

In certain embodiments, Compound No. 814907 is characterized by the following chemical notation: mCes mCeo Ges Tes Tes Tds Tds mCds Tds Tds Ads mCds mCds Aes mCeo mCes mCes Te; wherein,
A = an adenine,
mC = a 5-methylcytosine,
G = a guanine,
T = a thymine,
e = a 2'-MOE nucleoside,
d = a 2'-deoxynucleoside,
s = a phosphorothioate internucleoside linkage, and
o = a phosphodiester internucleoside linkage.

In certain embodiments, Compound No. 814907 is characterized by the following chemical structure:

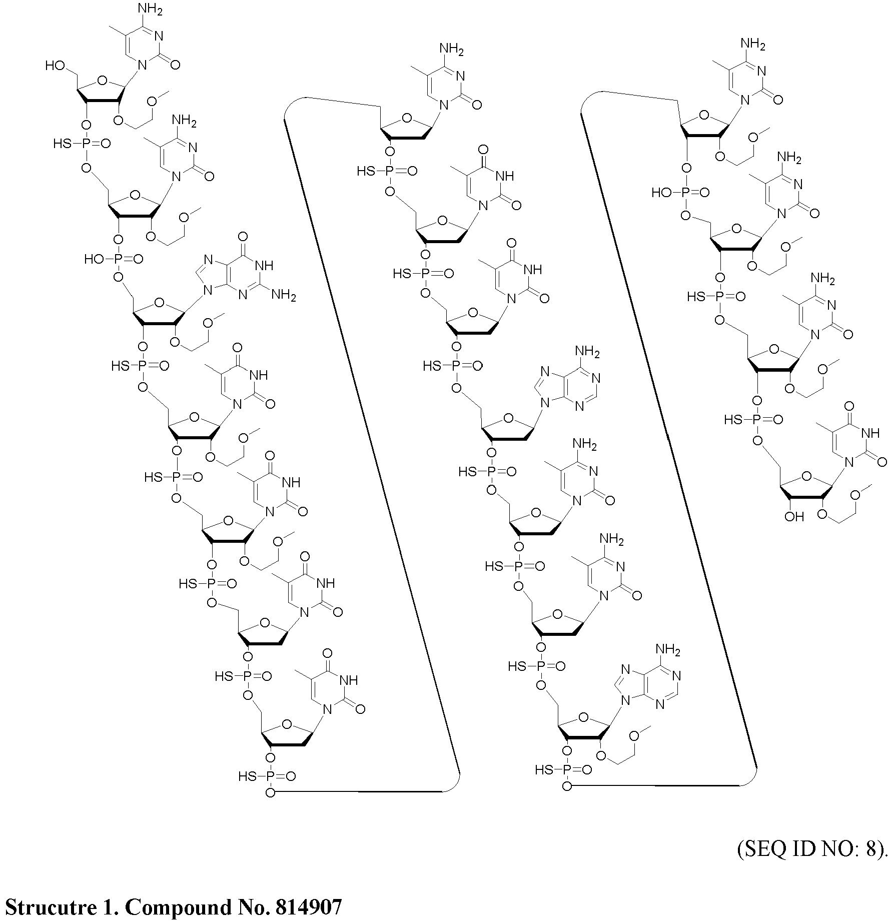

### VIII. Certain Benchmarks

In certain embodiments, Compound No. 623782 (characterized hereinbelow in Example 1), first described in WO 2015/010135, is a benchmark. Compound No. 623782 was a top performer among the compounds described in WO 2015/010135 in terms of potency, efficacy, and tolerability. Compound No. 623782 is provided as a benchmark to demonstrate the superior efficacy and tolerability of Compound No. 814907 (characterized hereinbelow in Example 1) as compared to Compound No. 623782 in comparative studies described hereinbelow in Example 1 and Example 2.

As demonstrated in Example 1, Compound No. 814907 achieved an ED₅₀ of 25 µg in Tau transgenic mice treated with 30 µg, 100 µg, 500 µg, whereas Compound No. 623782 achieved an ED₅₀ of 94 µg in Tau transgenic mice treated with 10 µg, 30 µg, 100 µg, 300 µg, 700 µg. Thus, Compound No. 814907 is more efficacious than Compound No. 623782.

As demonstrated in Example 2, administration of Compound No. 814907 to wild-type mice resulted in no Purkinje cell loss, whereas administration of Compound No. 623782 resulted in Purkinje cell loss in calbindin stained cerebellum sections in 3 of 11 animals. Therefore, Compound No. 814907 is more tolerable than Compound No. 623782.

### Disclosure

While certain compounds, compositions and methods described herein have been described with specificity in accordance with certain embodiments, the following examples serve only to illustrate the compounds described herein and are not intended to limit the same.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH in place of one 2'-H of DNA) or as an RNA having a modified base (thymine (methylated uracil) in place of a uracil of RNA). Accordingly, nucleic acid sequences described herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified nucleobases, such as "AT^{m}CGAUCG," wherein ^{m}C indicates a cytosine base comprising a methyl group at the 5-position.

Certain compounds described herein (e.g., modified oligonucleotides) have one or more asymmetric center and thus give rise to enantiomers, diastereomers, and other stereoisomeric configurations that may be defined, in terms of absolute stereochemistry, as (*R*) or (*S*), as α or β such as for sugar anomers, or as (D) or (L), such as for amino acids, etc. Compounds described herein that are drawn or described as having certain stereoisomeric configurations include only the indicated compounds. Compounds described herein that are drawn or described with undefined stereochemistry include all such possible isomers, including their stereorandom and optically pure forms, unless specified otherwise. Likewise, tautomeric forms of the compounds herein are also included unless otherwise indicated. Unless otherwise indicated, oligomeric compounds and modified oligonucleotides described herein are intended to include corresponding salt forms.

The compounds described herein include variations in which one or more atoms are replaced with a non-radioactive isotope or radioactive isotope of the indicated element. For example, compounds herein that comprise hydrogen atoms encompass all possible deuterium substitutions for each of the ¹H hydrogen atoms. Isotopic substitutions encompassed by the compounds herein include but are not limited to: ²H or ³H in place of ¹H, ¹³C or ¹⁴C in place of ¹²C, ¹⁵N in place of ¹⁴N, ¹⁷O or ¹⁸O in place of ¹⁶O, and ³³S, ³⁴S, ³⁵S, or ³⁶S in place of ³²S. In certain embodiments, non-radioactive isotopic substitutions may impart new properties on the oligomeric compound that are beneficial for use as a therapeutic or research tool. In certain embodiments, radioactive isotopic substitutions may make the compound suitable for research or diagnostic purposes such as imaging.

### EXAMPLES

The following examples illustrate certain embodiments of the present disclosure and are not limiting. Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

### Example 1: Effects of modified oligonucleotides on human Tau mRNA in transgenic mice

The modified oligonucleotides shown in the table below are 100% complementary to human Tau pre-mRNA (GENBANK Accession No. NT_010783.14, truncated from nucleotides 2624000 to 2761000, designated herein as SEQ ID NO: 1). The efficacies of the modified oligonucleotides were tested in human Tau transgenic mice (Duff et al., Neurobiology of Disease 7:87-98, 2000). Each mouse received a dose of a modified oligonucleotide listed in the table below, or PBS vehicle only, by ICV bolus injection. Each treatment group consisted of 2 to 4 mice. Several days after oligonucleotide administration, the mice were sacrificed and tissues were collected. RNA was extracted from the cortex and analyzed by RT-qPCR in order to determine human Tau mRNA levels. Primer probe set RTS3104, with the following sequences, was used: forward primer 5'-AAGATTGGGTCCCTGGACAAT-3', designated herein as SEQ ID NO: 5; reverse primer 5'-AGCTTGTGGGTTTCAATCTTTTTATT-3', designated herein as SEQ ID NO: 6; probe 5'-CACCCACGTCCCTGGCGGA-3', designated herein as SEQ ID NO: 7. Results are presented in the table below as the average percent inhibition of human Tau mRNA expression for each treatment group compared to the vehicle treated group. The half maximal effective dose (ED₅₀) for each modified oligonucleotide was calculated using nonlinear regression analysis.

**Table 1**

| **Percent inhibition of human Tau mRNA levels in hTau mice** | | | | | |
|---|---|---|---|---|---|
| Compound No. | Sequence | Dose (µg) | Human Tau mRNA (% inhibition) | ED₅₀ (µg) | SEQ ID NO. |
| 623782 | 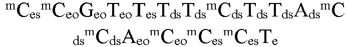 | 10 | 10 | 94 | 8 |
| | | 30 | 17 | | |
| | | 100 | 54 | | |
| | | 300 | 79 | | |
| | | 700 | 90 | | |
| 814907 | 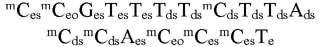 | 30 | 48 | 25 | 8 |
| | | 100 | 79 | | |
| | | 500 | 88 | | |

| | | | | | |
|---|---|---|---|---|---|
| Subscripts: "e" represents a 2'-MOE nucleoside; "d" represents a 2'-deoxynucleoside; "o" represents a phosphodiester internucleoside linkage; and "s" represents a phosphorothioate internucleoside linkage. Superscript "m" preceding a "C" indicates that the cytosine is a 5-methylcytosine. | | | | | |

### Example 2: Tolerability of modified oligonucleotides targeting human Tau

The tolerability of the modified oligonucleotides described in Example 1 was tested in wild type mice. Each mouse received a 700 µg injection of Compound No. 623782 or Compound No. 814907 at 700 µg dose, or PBS vehicle alone. Eight weeks after the modified oligonucleotide administration, the mice were sacrificed, and tissues were collected. Histopathology was performed on sections of cerebellum using H&E, IBA1, GFAP, and calbindin stains, and no abnormality relative to vehicle treated mice was observed for Compound No. 814907 treated mice. In a comparable experiment, Purkinje cell loss was observed in calbindin stained cerebellum sections in 3 of 11 animals treated with Compound No. 623782.

### Example 3: Effect of Compound No. 814907 in cynomolgous monkey following repeat-dose intrathecal injection for 13-weeks

Cynomolgus monkeys were treated with Compund No. 814907 to determine the local and systemic tolerability and pharmacokinetics at three dose levels, following repeat intrathecal lumbar bolus injections for 13 weeks. Compound No. 814907 shares complete sequence homology to the monkey Tau mRNA and has demonstrated pharmacologic activity in this species.

### Treatment

Cynomolgus monkeys ranging in age from 2-4 years were treated vehicle control (n=12) or Compound No. 814907 intrathecally (between L3-L4). Animals were dosed on Days 1, 14, 28, 56, and 84. Treatment groups received 4 mg (n=6), 12 mg (n=6), or 35 mg (n=14) of Compound No. 814907. Animals were sacrificed either on day 98 or 155 (4 animals from the vehicle control group and 4 animals from the 35 mg treatment group).

### Tolerability

Assessment of tolerability was based on clinical observations, body weights, food consumption, physical and neurological examinations, neurobehavioral observations (modified Irwin test (Irwin, 1968)), electrocardiogram (ECG) and blood pressure evaluation, ophthalmology, coagulation, hematology, clinical chemistry (blood and cerebral spinal fluid [CSF]), cell count (CSF only), blood gas evaluation, urine analysis, and anatomic pathology evaluations. Complete necropsies were performed with a recording of any macroscopic abnormality. Organ weights were taken and microscopic examinations were conducted. Blood was collected for complement analysis. In addition, blood, CSF, and tissues (at necropsy) were collected for toxicokinetic evaluations.

Intrathecal administration of 4 mg, 12 mg, or 35 mg Compound No. 814907 for 13 weeks (bi-weekly for the first month, then monthly thereafter) showed good local and systemic tolerability in male and female cynomolgus monkeys at all tested dosing regimens.

### Activity

Brain and spinal cord tissue was analyzed for inhibition of cynomolgous monkey Tau mRNA. Brain slices and spinal cord samples were collected and flash frozen in liquid nitrogen and stored frozen (-60°C to - 90°C). At time of sampling, 2 mm biopsy punches were used to collect samples for RNA analysis from the frozen brain slices. Punches were taken from multiple spinal cord and brain regions.

Total RNA from brain and spinal cord samples from cynomolgous monkeys treated with control or Compound No. 814907 were purified using a Life Technologies mini-RNA purification kit and subjected to real time PCR analysis. Monkey primer probe set rhMATPT LTS01278 (forward sequence AGGACAGAGTGCAGTCGAAGATC, designated herein as SEQ ID NO: 9; reverse sequence AGGTCAGCTTGTGGGTTTCAA, designated herein as SEQ ID NO: 10; probe sequence CACCCATGTCCCTGGCGGAGG, designated herein as SEQ ID NO: 11) was used to measure RNA levels. Tau RNA was then normalized to the housekeeping gene Cyclophilin A. All qPCR reactions were run in triplicate. Data is reported relative to mRNA levels in animals treated with artificial CSF.

As shown in the Table below, there was a significant and dose responsive decrease in Tau RNA levels in spinal cord and multiple CNS regions after treatment with Compound No. 814907, as compared to control treated monkeys.

**Table 2**

| **Percent inhibition of cynomolgous Tau mRNA levels in cynomolgus monkeys** | | | | | | | |
|---|---|---|---|---|---|---|---|
| Treatment | Brain Regions | | | | | | |
| | Thoracic Spinal Cord | Lumbar Spinal Cord | Cortical Spinal Cord | Frontal Cortex | Temporal Cortex | Hippocampus | Pons |
| aCSF | 0 | 0 | 0 | 0 | 0 | 0 | 0 |
| 4 mg 814907 | 51 | 63 | 31 | 41 | 29 | 18 | 29 |
| 12 mg 814907 | 60 | 60 | 47 | 60 | 56 | 52 | 43 |
| 35 mg 814907 | 58 | 71 | 60 | 77 | 70 | 74 | 66 |

### Example 4: Phase I-IIa HumanClinical Trial with Compound No. 814907

Multiple ascending doses of Compound No. 814907 are evaluated in a randomized, double-blind, placebo-controlled study to evalulate the safety, tolerability, pharmokinetics and pharmacodynamics in patients with mild Alzheimer's Disease (AD) aged 50-74 years of age. Eligible patients will have CSF AD biomarker evidence of amyloid and tau pathology in addition to meeting clinical criteria for AD. Four ascending dose level cohorts of mild AD patients will be enrolled sequentially and randomized 3:1 to receive Compound No. 814907 or placebo. Each patient will receive 4 doses of Compound No.814907 or placebo with a 28 day interval between doses. Patients will receive 4 intrathecal (IT) bolus doses of Compound No 814907 at 4 week intervals during the 3 month treatment period (on Days 1, 29, 57, 85). Each dose of Compound No. 814907 or placebo will be administered as a single 20 mL IT bolus injection. Administration will be via lumbar puncture using a small gauge needle inserted into the L3/L4 space.

### Safety and Tolerability Evaluations

Patient safety will be monitored closely during the study. Safety and tolerability evaluations include: physical examination and standard neurological assessment (including fundi), vital signs (HR, BP, orthostatic changes, weight), ECG, AEs and concomitant medications, Columbia Suicide Severity Rating Scale (C-SSRS), CSF safety labs (cell counts, protein, glucose), plasma laboratory tests (clinical chemistry, hematology), urinalysis, and neuroimaging assessments will be conducted using a 3T MRI scanner. Clinical and volumetric neuroimaging measures will be used to monitor for unexpected deterioration.

### Pharmacokinetic Evaluations

A CSF sample will be collected pre-dose on each administration day (Days 1, 29, 57, 85) and during the post-treatment period for PK analyses.

### Exploratory Evaluations

Biochemical, neuroimaging, functioning/ability to perform activities of daily living, cognitive, and neuropsychiatric parameters will be evaluated.

Biochemical parameters include potential CSF and blood/plasma biomarkers, including target engagement, neuronal and synaptic injury markers, innate immune activation markers, complement components, and lipid-related biomarkers.

Neuroimaging paramers include structural MRI (hippocampal, whole brain, and ventricular volumes), Arterial Spin Labelling (ASL), diffusion tensor imaging (DTI), and FDG-PET (Cohorts C and D only).

Functioning/ability to perform activities of daily living parameters include evaluation by Functional Activities Questionnaire (FAQ).

Cognitive parameters include evaluation by Repeatable Battery for the Assessment of Neuropsychological Status (RBANS) and Mini-mental state examination (MMSE) Neuropsychiatric parameters include evaluation by Neuropsychiatric Inventory - Questionnaire (NPI-Q)

## Claims

1. A modified oligonucleotide according to the following formula: 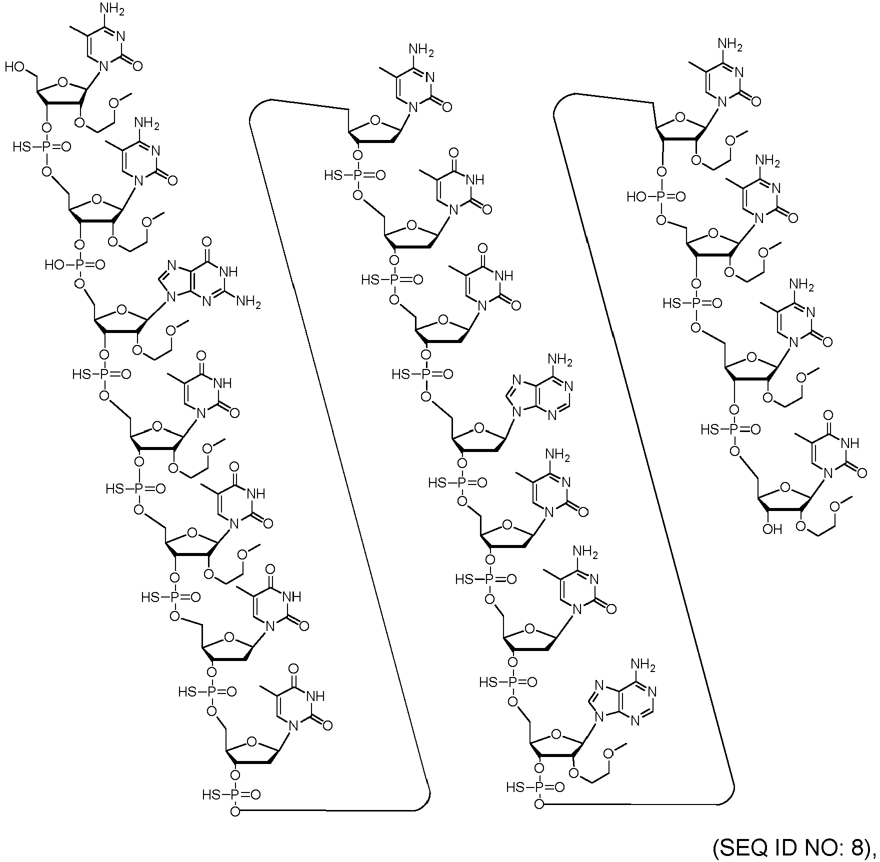 or a salt thereof, for use in a method of treating a neurodegenerative disease associated with tau in a human subject in need thereof.

2. The salt of the modified oligonucleotide for use of claim 1, wherein the salt includes a sodium salt and/or a potassium salt.

3. A modified oligonucleotide for use in a method of treating a neurodegenerative disease associated with tau in a human subject in need thereof, wherein the modified oligonucleotide is a gapmer consisting of a 5' wing segment, a central gap segment, and a 3' wing segment, wherein:
the 5' wing segment consists of five 2'-MOE nucleosides,
the central gap segment consists of eight 2'-deoxynucleosides, and
the 3' wing segment consists of five 2'-MOE nucleosides;
wherein the modified oligonucleotide has the nucleobase sequence 5'-CCGTTTTCTTACCACCCT-3' (SEQ ID NO: 8),
wherein each cytosine is a 5-methylcytosine; and
wherein the internucleoside linkages of the modified oligonucleotide are, from 5' to 3', sossssssssssssoss, wherein each s is a phosphorothioate linkage and each o is a phosphodiester linkage.

4. A compound comprising a modified oligonucleotide for use in a method of treating a neurodegenerative disease associated with tau in a human subject in need thereof, wherein the modified oligonucleotide is a gapmer consisting of a 5' wing segment, a central gap segment, and a 3' wing segment, wherein:
the 5' wing segment consists of five 2'-MOE nucleosides,
the central gap segment consists of eight 2'-deoxynucleosides, and
the 3' wing segment consists of five 2'-MOE nucleosides;
wherein the modified oligonucleotide has the nucleobase sequence 5'-CCGTTTTCTTACCACCCT-3' (SEQ ID NO: 8),
wherein each cytosine is a 5-methylcytosine; and
wherein the internucleoside linkages of the modified oligonucleotide are, from 5' to 3', sossssssssssssoss, wherein each s is a phosphorothioate linkage and each o is a phosphodiester linkage.

5. The modified oligonucleotide for use of claim 3, or the compound for use of claim 4, wherein the modified oligonucleotide is linked to a conjugate group.

6. The modified oligonucleotide or salt thereof for use of any one of claims 1-3 and 5, or the compound for use of claim 4 or claim 5, wherein the modified oligonucleotide, salt, or compound reduces the amount or activity of Tau messenger RNA (mRNA).

7. The modified oligonucleotide or salt thereof for use of any one of claims 1-3 and 5-6, or the compound for use of any one of claims 4-6, wherein the neurodegenerative disease is a tauopathy.

8. The modified oligonucleotide or salt thereof for use of any one of claims 1-3 and 5-6, or the compound for use of any one of claims 4-6, wherein the neurodegenerative disease is Alzheimer's Disease, Fronto-temporal Dementia (FTD), FTDP-17, Progressive Supranuclear Palsy (PSP), Chronic Traumatic Encephalopathy (CTE), Corticobasal Ganglionic Degeneration (CBD), Epilepsy, or Dravet's Syndrome.

9. The modified oligonucleotide, salt, or compound for use of claim 8, wherein the neurodegenerative disease is Alzheimer's Disease.

10. The modified oligonucleotide, salt, or compound for use of claim 9, wherein the Alzheimer's Disease is mild Alzheimer's Disease.

11. The modified oligonucleotide, salt, or compound for use of claim 8, wherein the neurodegenerative disease is Fronto-temporal Dementia (FTD).

12. The modified oligonucleotide, salt, or compound for use of claim 8, wherein the neurodegenerative disease is Progressive Supranuclear Palsy (PSP).

13. The modified oligonucleotide, salt, or compound for use of any preceding claim, wherein the modified oligonucleotide, salt, or compound is administered by intrathecal administration.

## Patentansprüche

1. Modifiziertes Oligonukleotid gemäß der folgenden Formel: 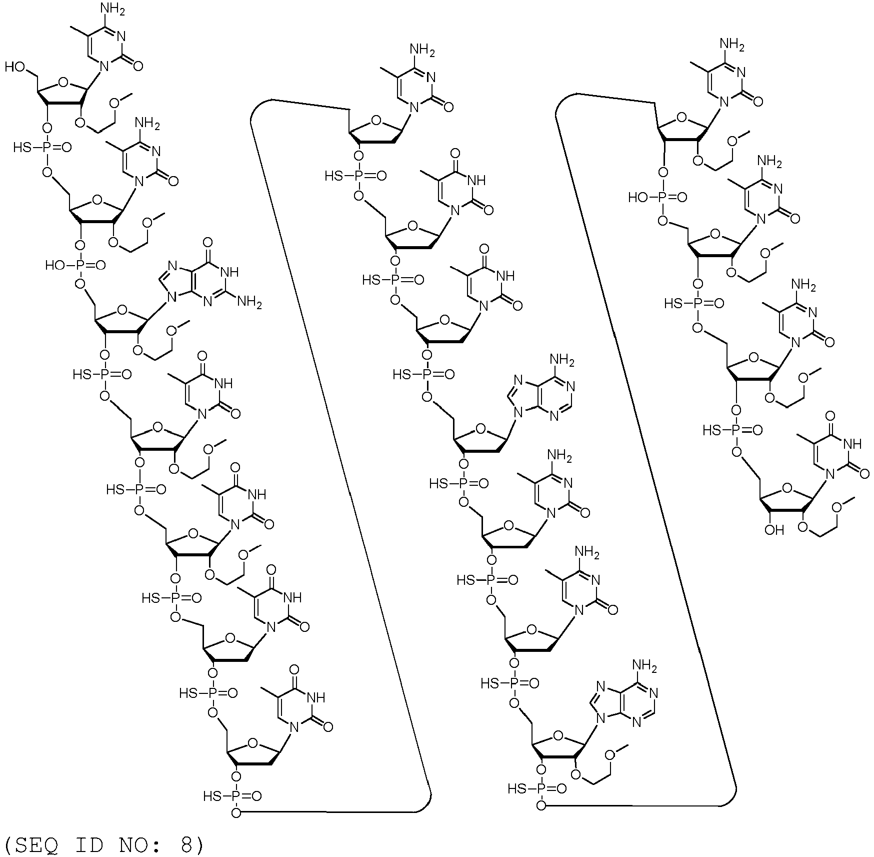 oder ein Salz davon zur Verwendung bei einem Verfahren zur Behandlung einer mit tau assoziierten neurodegenerativen Erkrankung bei einem behandlungsbedürftigen menschlichen Individuum.

2. Salz des modifizierten Oligonukleotids zur Verwendung nach Anspruch 1, wobei das Salz ein Natriumsalz und/oder ein Kaliumsalz umfasst.

3. Modifiziertes Oligonukleotid zur Verwendung bei einem Verfahren zur Behandlung einer mit tau assoziierten neurodegenerativen Erkrankung bei einem behandlungsbedürftigen menschlichen Individuum, wobei es sich bei dem modifizierten Oligonukleotid um ein Gapmer handelt, das aus einem 5'-Wing-Segment, einem zentralen Gap-Segment und einem 3'-Wing-Segment besteht, wobei:
das 5'-Wing-Segment aus fünf 2'-MOE-Nukleosiden besteht, das zentrale Gap-Segment aus acht 2'-Desoxynukleosiden besteht und
das 3'-Wing-Segment aus fünf 2'-MOE-Nukleosiden besteht, wobei das modifizierte Oligonukleotid die Nukleobasensequenz 5'-CCGTTTTCTTACCACCCT-3' (SEQ ID NO: 8) aufweist,
wobei es sich bei den Cytosinen jeweils um ein 5-Methylcytosin handelt; und
wobei es sich bei den Internukleosidverknüpfungen des modifizierten Oligonukleotids um, von 5' nach 3', sossssssssssssoss handelt, wobei s jeweils für eine Phosphorthioatverknüpfung und o jeweils für eine Phosphodiesterverknüpfung steht.

4. Verbindung, umfassend ein modifiziertes Oligonukleotid, zur Verwendung bei einem Verfahren zur Behandlung einer mit tau assoziierten neurodegenerativen Erkrankung bei einem behandlungsbedürftigen menschlichen Individuum,
wobei es sich bei dem modifizierten Oligonukleotid um ein Gapmer handelt, das aus einem 5'-Wing-Segment, einem zentralen Gap-Segment und einem 3'-Wing-Segment besteht,
wobei:
das 5'-Wing-Segment aus fünf 2'-MOE-Nukleosiden besteht,
das zentrale Gap-Segment aus acht 2'-Desoxynukleosiden besteht und
das 3'-Wing-Segment aus fünf 2'-MOE-Nukleosiden besteht, wobei das modifizierte Oligonukleotid die Nukleobasensequenz 5'-CCGTTTTCTTACCACCCT-3' (SEQ ID NO: 8) aufweist,
wobei es sich bei den Cytosinen jeweils um ein 5-Methylcytosin handelt; und
wobei es sich bei den Internukleosidverknüpfungen des modifizierten Oligonukleotids um, von 5' nach 3', sossssssssssssoss handelt, wobei s jeweils für eine Phosphorthioatverknüpfung und o jeweils für eine Phosphodiesterverknüpfung steht.

5. Modifiziertes Oligonukleotid zur Verwendung nach Anspruch 3 oder Verbindung zur Verwendung nach Anspruch 4, wobei das modifizierte Oligonukleotid mit einer Konjugatgruppe verknüpft ist.

6. Modifiziertes Oligonukleotid oder Salz davon zur Verwendung nach einem der Ansprüche 1-3 und 5 oder Verbindung zur Verwendung nach Anspruch 4 oder Anspruch 5, wobei durch das modifizierte Oligonukleotid, das Salz oder die Verbindung die Menge oder Aktivität von Tau-mRNA (messenger RNA) verringert wird.

7. Modifiziertes Oligonukleotid oder Salz davon zur Verwendung nach einem der Ansprüche 1-3 und 5-6 oder Verbindung zur Verwendung nach einem der Ansprüche 4-6, wobei es sich bei der neurodegenerativen Erkrankung um eine Tauopathie handelt.

8. Modifiziertes Oligonukleotid oder Salz davon zur Verwendung nach einem der Ansprüche 1-3 und 5-6 oder Verbindung zur Verwendung nach einem der Ansprüche 4-6, wobei es sich bei der neurodegenerativen Erkrankung um Morbus Alzheimer, Frontotemporale Demenz (FTD), FTDP-17, Progressive supranukleäre Blickparese (PSP), Chronischtraumatische Enzephalopathie (CTE), Corticobasalganglionäre Degeneration (CBD), Epilepsie oder Dravet-Syndrom handelt.

9. Modifiziertes Oligonukleotid, Salz oder Verbindung zur Verwendung nach Anspruch 8, wobei es sich bei der neurodegenerativen Erkrankung um Morbus Alzheimer handelt.

10. Modifiziertes Oligonukleotid, Salz oder Verbindung zur Verwendung nach Anspruch 9, wobei es sich bei dem Morbus Alzheimer um eine milde Form handelt.

11. Modifiziertes Oligonukleotid, Salz oder Verbindung zur Verwendung nach Anspruch 8, wobei es sich bei der neurodegenerativen Erkrankung um Frontotemporale Demenz (FTD) handelt.

12. Modifiziertes Oligonukleotid, Salz oder Verbindung zur Verwendung nach Anspruch 8, wobei es sich bei der neurodegenerativen Erkrankung um Progressive supranukleäre Blickparese (PSP) handelt.

13. Modifiziertes Oligonukleotid, Salz oder Verbindung zur Verwendung nach einem vorhergehenden Anspruch, wobei das modifizierte Oligonukleotid, das Salz oder die Verbindung über intrathekale Verabreichung verabreicht wird.

## Revendications

1. Oligonucléotide modifié selon la formule suivante : 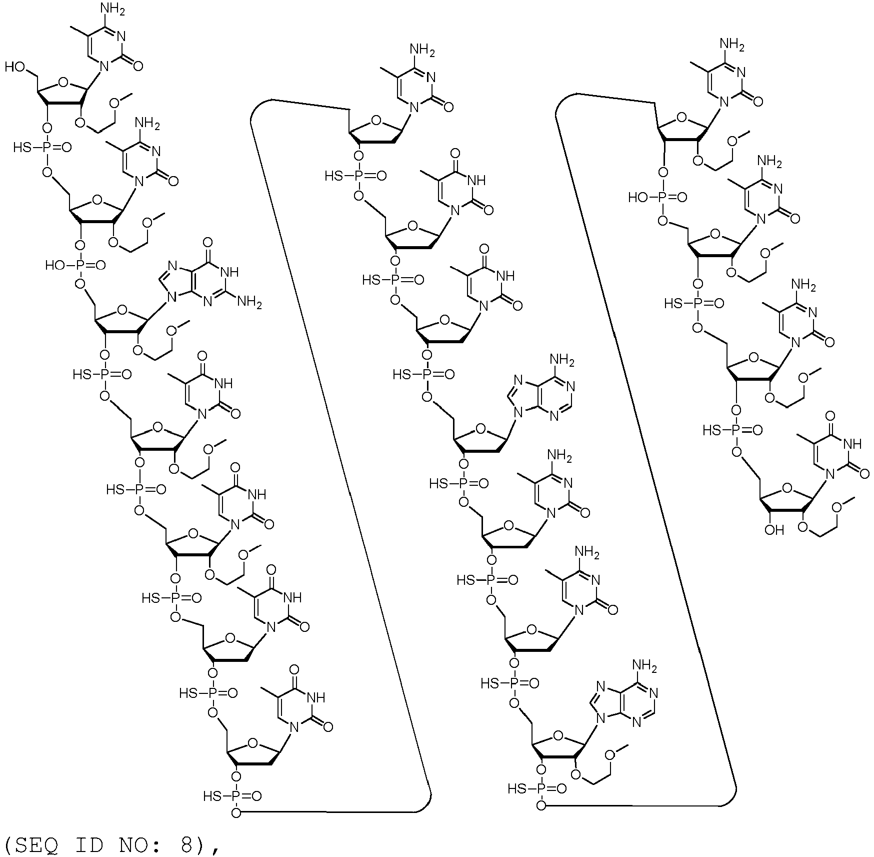 ou sel de celui-ci, pour utilisation dans un procédé de traitement d'une maladie neurodégénérative associée à tau chez un sujet humain en ayant besoin.

2. Sel de l'oligonucléotide modifié pour utilisation selon la revendication 1, dans lequel le sel est un sel de sodium et/ou un sel de potassium.

3. Oligonucléotide modifié destiné à être utilisé dans un procédé de traitement d'une maladie neurodégénérative associée à tau chez un sujet humain qui en a besoin, dans lequel l'oligonucléotide modifié est un gapmère constitué d'un segment d'aile en 5', d'un segment de brèche central et d'un segment d'aile en 3', dans lequel :
le segment d'aile en 5' est constitué de cinq nucléosides 2'-MOE,
le segment de brèche central est constitué de huit 2'-désoxynucléosides, et
le segment d'aile en 3' est constitué de cinq nucléosides 2'-MOE ;
dans lequel l'oligonucléotide modifié a la séquence de nucléobases 5'-CCGTTTTCTTACCACCCT-3' (SEQ ID NO: 8),
dans lequel chaque cytosine est une 5-méthylcytosine ; et
dans lequel les liaisons internucléosidiques de l'oligonucléotide modifié sont, de 5' à 3', sossssssssssssoss, chaque s étant une liaison phosphorothioate et chaque o étant une liaison phosphodiester.

4. Composé comprenant un oligonucléotide modifié pour utilisation dans un procédé de traitement d'une maladie neurodégénérative associée à tau chez un sujet humain en ayant besoin,
dans lequel l'oligonucléotide modifié est un gapmère constitué d'un segment d'aile en 5', un segment de brèche central et un segment d'aile en 3', dans lequel :
le segment d'aile en 5' est constitué de cinq nucléosides 2'-MOE,
le segment de brèche central est constitué de huit 2'-désoxynucléosides, et
le segment d'aile en 3' est constitué de cinq nucléosides 2'-MOE ;
dans lequel l'oligonucléotide modifié a la séquence de nucléobases 5'-CCGTTTTCTTACCACCCT-3' (SEQ ID NO: 8),
dans lequel chaque cytosine est une 5-méthylcytosine ; et
dans lequel les liaisons internucléosidiques de l'oligonucléotide modifié sont, de 5' à 3', sossssssssssssoss, chaque s étant une liaison phosphorothioate et chaque o étant une liaison phosphodiester.

5. Oligonucléotide modifié pour utilisation selon la revendication 3, ou composé pour utilisation selon la revendication 4, dans lequel l'oligonucléotide modifié est lié à un groupe conjugué.

6. Oligonucléotide modifié ou sel de celui-ci pour utilisation selon l'une quelconque des revendications 1-3 et 5, ou composé pour utilisation selon la revendication 4 ou la revendication 5, dans lequel l'oligonucléotide modifié, le sel ou le composé réduit la quantité ou l'activité de l'ARN messager (ARNm) Tau.

7. Oligonucléotide modifié ou sel de celui-ci pour utilisation selon l'une quelconque des revendications 1-3 et 5-6, ou composé pour utilisation selon l'une quelconque des revendications 4-6, dans lequel la maladie neurodégénérative est une tauopathie.

8. Oligonucléotide modifié ou sel de celui-ci pour utilisation selon l'une quelconque des revendications 1-3 et 5-6, ou composé pour utilisation selon l'une quelconque des revendications 4-6, dans lequel la maladie neurodégénérative est la maladie d'Alzheimer, la démence fronto-temporale (DFT), le FTDP-17, la paralysie supranucléaire progressive (PSP), l'encéphalopathie traumatique chronique (ETC), la dégénérescence ganglionnaire corticobasale (DCC), l'épilepsie ou le syndrome de Dravet.

9. Oligonucléotide modifié, sel ou composé pour utilisation selon la revendication 8, dans lequel la maladie neurodégénérative est la maladie d'Alzheimer.

10. Oligonucléotide modifié, sel ou composé pour utilisation selon la revendication 9, dans lequel la maladie d'Alzheimer est la maladie d'Alzheimer légère.

11. Oligonucléotide modifié, sel ou composé pour utilisation selon la revendication 8, dans lequel la maladie neurodégénérative est la démence fronto-temporale (DFT).

12. Oligonucléotide modifié, sel ou composé pour utilisation selon la revendication 8, dans lequel la maladie neurodégénérative est la paralysie supranucléaire progressive (PSP).

13. Oligonucléotide modifié, sel ou composé pour utilisation selon l'une quelconque des revendications précédentes, dans lequel l'oligonucléotide, le sel ou le composé modifié est administré par administration intrathécale.
